# EUROPÄISCHE PATENTSCHRIFT

(11) **EP 0 526 800 B1**
(45) Veröffentlichungstag und Bekanntmachung des Hinweises auf die Patenterteilung: **08.01.1997**
(21) Anmeldenummer: 92112512.6
(22) Anmeldetag: 22.07.1992
(51) Int. Cl.: C08F 226/00, C08F 220/34, A61K 7/48, A61K 7/06

(54) **Polymerisate aus ethylenisch ungesättigten, N-haltigen Verbindungen, polymerisiert in Gegenwart von Monosacchariden, Oligosacchariden, Polysacchariden oder deren Derivaten**
Polymers from N-containing monomers polymerised in the presence of monosaccharids, oligosaccharids or polysaccharids and derivatives
Polymères de composés contenant de l'azote polymérisés en présence de monosaccharides, oligosaccharides et polysaccharides et leurs dérivés

(30) Priorität: 03.08.1991 DE 4125752
(43) Veröffentlichungstag der Anmeldung: 10.02.1993
(73) Patentinhaber: BASF Aktiengesellschaft, 67063 Ludwigshafen (DE)
(72) Erfinder: Meyer, Harald, Dr., W-6705 Deidesheim (DE); Denzinger, Walter, W-6720 Speyer (DE); Sanner, Axel, Dr., W-6710 Frankenthal (DE); Reinhardt, Rolf-Dieter, Dr., W-6711 Laumersheim (DE); Frosch, Franz, Dr., W-6702 Bad Duerkheim (DE); Raubenheimer, Hans-Juergen, W-6834 Ketsch (DE)

(56) Entgegenhaltungen:
- EP-A- 0 405 917
- EP-A- 0 408 099
- EP-A- 0 441 197
- US-A- 4 131 576
- US-A- 4 803 071
- US-A- 4 831 097
- DATABASE WPIL Section Ch, Week 8331, Derwent Publications Ltd., London, GB; Class A96, AN 83-725067 & JP-A-58 038 206 (KANEBO KK) 5. Maerz 1983

## Beschreibung

Die vorliegende Erfindung betrifft ein Verfahren zur Herstellung von Polymerisaten aus ethylenisch ungesättigten, N-haltigen Verbindungen, welche in Gegenwart von Monosacchariden, Oligosacchariden, Polysacchariden oder deren Derivaten polymerisiert werden.

Auf dem Gebiet der Kosmetik finden Polymerisate oder Copolymerisate aus beispielsweise N-Vinyllactamen, N-Vinylimidazolen oder N-Vinylimidazolinium-Salzen sowie Dialkylaminoalkyl-(meth)acrylsäureestern bzw. -(meth)acrylsäureamiden oder deren Quaternisierungsprodukten schon seit längerem Verwendung.

So sind aus der DE-C 36 17 069 (1) Homopolymere von 3-Alkylvinylimidazoliumchloriden und Copolymere von Vinyllactamen mit 3-Alkylvinylimidazoliumchloriden bekannt, die als Haarkonditioniermittel Anwendung finden und durch radikalische Polymerisation in wäßrigen und wäßrigalkoholischen Lösungen bei 40 bis 100°C gewonnen werden können.

Aus der US-A 3 910 862 (2) sind quaternäre Copolymere bekannt, die aus 20 bis 95 Mol-% Vinylpyrrolidon, 5 bis 80 Mol-% Dialkylaminoalkylacrylaten oder -methacrylaten und 0 bis 50 Mol-% eines mit Vinylpyrrolidon copolymerisierbaren Monomeren bestehen, als wäßrige, alkoholische oder wäßrig-alkoholische Lösung vorliegen und Anwendung in kosmetischen Zusammensetzungen finden.

In der WO 90/01920 (3) werden Copolymere von N-(Dialkylaminoalkyl)acrylamiden oder -methacrylamiden oder deren Quaternisierungsprodukten mit Vinyllactamen beschrieben. Die erhaltenen wasserlöslichen Copolymeren werden zur Pflege des Haares und der Haut angewendet.

Diese aus dem Stand der Technik bekannte Polymeren weisen jedoch eine Reihe von Nachteilen auf. Beispielsweise ist bei ihnen die Wasseraufnahme noch zu hoch und die Anionentensid-Verträglichkeit noch zu gering. In haarkosmetischen Zubereitungen sind beispielsweise die Naßkämmbarkeit der Haare und generell die Pflegewirkung noch verbesserungsbedürftig.

Komplexe von Poly-N-Vinylpyrrolidon mit Iod, sogenannte Iodophore, finden breite Anwendung als Feindesinfektionsmittel. Lagerstabile Komplexe werden wie aus der USP 4 027 083 bekannt nur dann erhalten, wenn das Polymere in aufwendiger Weise in wasserfreien Lösemitteln hergestellt wird, die nach der Polymerisation weitgehend entfernt werden müssen.
Aus der DE-A 27 42 595 sind Pfropfcopolymerisate aus Stärke und N-Vinylpyrrolidon bekannt, die durch Polymerisation mit dem Redoxstarter Mn³⁺ erhalten werden. Schwermetallhaltige Produkte weisen den Nachteil auf, daß sie in vielen Einsatzgebieten wegen des Schwermetallgehaltes nicht einsetzbar sind.

In der USP 4 131 576 sind Pfropfcopolymere aus Stärke und wasserlöslichen Monomeren beschrieben, die durch Polymerisation in Suspension erhalten und als Feststoff isoliert werden. Bekanntermaßen sind die Pfropfgrade in so durchgeführten Polymerisationen sehr niedrig.

Aufgabe der vorliegenden Erfindung war es, der Technik neue Mittel zur Verfügung zu stellen, die die Nachteile des Standes der Technik nicht mehr aufweisen.

Demgemäß wurde ein Verfahren zur Herstellung von Polymerisaten aus ethylenisch ungesättigten Verbindungen, die mindestens ein kovalent gebundenes N-Atom im Molekül enthalten, polymerisiert in Gegenwart von Monosacchariden, Oligosacchariden, Polysacchariden oder deren Derivaten, gefunden, welches dadurch gekennzeichnet ist, daß man
(A) Monomere oder Monomerenmischungen aus der Gruppe
   (a) N-Vinylimidazole, welche am heterocyclischen Ring durch bis zu drei C₁- bis C₁₂-Alkylrest substituiert sein und in N-quaternisierter Form oder in Salzform vorliegen können,
   (b) fünf- bis achtgliedrige N-Vinyllactame, welche am Ring durch bis zu drei C₁- bis C₁₂-Alkylreste substituiert sein können,
   (c) Acrylsäure- oder Methacrylsäure-dialkylaminoalkylester mit insgesamt bis zu 30 C-Atomen im Dialkylaminoalkyl-Rest, welche in N-quaternisierter Form oder in Salzform vorliegen können, und
   (d) N- (Dialkylaminoalkyl) -acrylsäure- oder -methacrylsäureamide mit insgesamt bis zu 30 C-Atomen in Dialkylaminoalkyl-Rest, welche in N-quaternisierter Form oder in Salzform vorliegen können,
   wobei als weitere Comonomere zu (A) noch
   (f) monoethylenisch ungesättigte C₃- bis C₁₀-Carbonsäuren und deren Alkalimetall-, Erdalkalimetall- oder Ammoniumsalze,
   (g) monoethylenisch ungesättigte C₃- bis C₁₀-Carbonsäureester sowie
   (h) in geringen Mengen mindestens zwei ethylenisch ungesättigte, nicht konjugierte Doppelbindungen im Molekül enthaltende Verbindungen
   vorhanden sein können, wobei entweder die Verbindungen (a) oder (b) jeweils allein oder Mischungen aus den Verbindungen (a) bis (d) gewünschtenfalls mit (f) bis (h) eingesetzt werden und wobei (h) maximal in einer Menge bis zu 5 Gew.-%, bezogen auf die Gesamtmenge aller Monomeren (A), verwendet wird, in Gegenwart von
(B) Monosacchariden, Oligosacchariden, Polysacchariden, thermisch oder mechanisch behandelten, oxidativ, hydrolytisch oder enzymatisch abgebauten Polysacchariden, oxidierten hydrolytisch oder enzymatisch abgebauten Polysacchariden, chemisch modifizierten Mono-, Oligo- und Polysacchariden oder Mischungen der genannten Verbindungen (B)
im Gewichtsverhältnis (A) : (B) von (95 bis 20) : (5 bis 80), insbesondere von (75 bis 40) : (25 bis 60), in wäßrigen Systemen durch Lösungspolymerisation radikalisch iniitiiert polymerisiert.

Beispiele für N-Vinylimidazole (a) sind das unsubstituierte 1-Vinylimidazol, 2-Methyl-1-vinylimidazol, 4-Methyl-1-vinylimidazol, 2,4-Di4-Methyl-1-vinylimidazol, oder 2-Ethyl-1-vinylimidazol.

Als geradkettige oder verzweigte C₁- bis C₁₂-Alkylreste als Substituenten am Ringsystem der Verbindungen (a) sowie auch als Substituenten bei der Verbindung (b) eignen sich vor allem Methyl, Ethyl, n-Propyl, iso-Propyl, n-Butyl, iso-Butyl, sec.-Butyl oder tert.-Butyl, daneben aber auch n-Pentyl, iso-Pentyl, n-Hexyl, n-Heptyl, n-Octyl, 2-Ethylhexyl, n-Nonyl, iso-Nonyl, n-Decyl, n-Undecyl, iso-Undecyl oder n-Dodecyl.

Beispiele für quaternisierte N-Vinylimidazole (a) sind 1-Vinylimidazoliumchlorid, 3-Methyl-1-vinylimidazoliumchlorid, 3-Methyl-1-vinylimidazoliumbromid, 3-Methyl-1-vinylimidazoliumchlorid, 2,3-Dimethyl-1-vinylimidazoliumchlorid, 3-n-Dodecyl-1-vinylimidazoliumchlorid, 3-Methyl-1-vinylimidazoliurnmethylsulfat, 3-Ethyl-1-vinylimidazoliumethylsulfat, 2,3-Dimethyl-1-vinylimidazoliumsulfat oder 2-Methyl-3-ethyl-1-vinylimidazoliumethylsulfat.

Als Gegenionen zu den quaternisierten Verbindungen oder Salzen (a), aber auch zu den quaternisierten Verbindungen oder Salzen (c), (d) und (e), können vor allem Halogenide, also Fluorid, Bromid, Iodid und insbesondere Chlorid, Acetat, Lactat, Hydrogensulfat, Sulfat, Mono- oder Dialkylsulfat, z.B. Methylsulfat oder Dimethylsulfat, Sulfonate oder Hydrogensulfit und Sulfit auftreten.

Geeignete Quaternisierungsmittel sind vor allem Dialkylsulfate, z.B. Dimethylsulfat, Diethylsulfat oder Methylsulfat, Alkylsulfonsäuren, z.B. Methylsulfonsäure oder Ethylsulfonsäure, Benzylhalogenide, z.B. Benzylchlorid, Benzylbromid oder Benzyliodid, Alkylhalogenide, z.B. Methylchlorid, Ethylbromid, Ethylchlorid, Octylchlorid oder Stearylchlorid, chlorhaltige Lactame, z.B. N-Chlormethylpyrrolidon oder N-Chlorethylcaprolactam, heterocyclische Sulfate, z.B. 1,2-Oxathiethan-2,2-dion, Lactone, z.B. β-Propiolacton oder γ-Butyrolacton sowie Kohlensäureester, z.B. Dimethylcarbonat, Methylethylcarbonat oder Diethylcarbonat.

Zur Überführung der basischen Monomeren (A) in ihre Salze verwendet man zweckmäßigerweise die entsprechenden Säuren, z.B. Salzsäure, Essigsäure oder Schwefelsäure.

Fünf- bis achtgliedrige N-Vinyllactame (b) sind beispielsweise 1-Vinylpyrrolidon, 1-Vinylcaprolactam, 1-Vinylpiperidon, 4-Methyl-1-vinylpyrrolidon, 3,5-Dimethyl-1-vinylcaprolactam oder 1-Vinyloctahydro-2-azocinon.

Als (Meth)acrylsäure-dialkylaminoalkylester (c) mit insgesamt bis zu 30 C-Atomen, insbesondere bis zu 22 C-Atomen im Dialkylaminoalkyl-Rest kommen z.B. in Betracht:

Dimethylaminoethylacrylat, Methylethylaminoethylacrylat, Dimethylaminomethylmethacrylat, Diethylaminoethylacrylat, Diethylaminoethylmethacrylat, Dimethylaminoethylacrylat, Dimethylaminoethylmethacrylat, Methylethylaminoethylmethacrylat, Dimethylaminobutylacrylat, Dimethylaminobutylmethacrylat, methylaminoamylmethacrylat, Diethylaminoamylmethacrylat, Dimethylaminohexylacrylat, Diethylaminohexylmethacrylat, Dimethylaminooctylacrylat, Dimethylaminooctylmethacrylat, Diethylaminooctylacrylat, Diethylaminooctylmethacrylat, Dimethylaminododecylmethacrylat, Diethylaminolaurylacrylat, Diethylaminolaurylmethacrylat, Dimethylaminostearylacrylat, Dimethylaminostearylmethacrylat, Diethylaminostearylacrylate, Diethylaminostearylmethacrylat, Di-tert.-butylaminoethylmethacrylat oder Di-tert.-butylaminoethylacrylat
sowie deren Quaternisierungsprodukte und deren Salze.

Als N-(Dialkylaminoalkyl)-(meth)acrylsäureamide (d) mit insgesamt bis zu 30 C-Atomen, insbesondere bis zu 22 C-Atomen im Dialkylaminoalkyl-Rest kommen z.B. in Betracht:

N-[3-(Dimethylamino)propyl]acrylamid, N-[3-(Dimethylamino) propyl] methacrylamid, N-[3-(Diethylamino)propyl]acrylamid, N-[3-(Diethylamino)propyl]methacrylamid, N-[12-(Dimethylamino)dodecyl]methacrylamid, N-[12-(Dimethylamino)dodecyl]acrylamid, N-[18-(Dimethylamino)octadecyl]methacrylamid, N-[18-(Dimethylamino)octadecyl]acrylamid, N-[8-(Dimethylamino) octyl] methacrylamid, N-[8-(Dimethylamino) octyl]acrylamid, N-[7-(Dimethylamino)heptyl]methacrylamid, N-[7-(Dimethylamino) heptyl] acrylamid, N-[14- (Dimethylamino)tetradecyl] methacrylamid, N-[14-(Dimethylamino)tetradecyl] acrylamid, N-[4-(Dimethylamino)butyl] methacrylamid, N-[4-(Dimethylamino)butyl]acrylamid, N-[4-(Diethylamino)butyl]methacrylamid, N-[4-(Diethylamino)butyl] acrylamid, N-[3-Methylethylamino)propyl] methacrylamid, N-[3-(Methylethylamino)propyl] acrylamid, N-[3-Methyl-5- (dimethylamino)pentyl]acrylamid, N-[3-Methyl-5-(dimethylamino)pentyl]methacrylamid, N-[2-Methyl-4-(dimethylamino)butyl]acrylamid oder N-[2-Methyl-4-(dimethylamino)butyl]methacrylamid
sowie deren Quaternisierungsprodukte und deren Salze.

Als Comonomere (f) können monoethylenisch ungesättigte C₃- bis C₁₀-Carbonsäuren sowie deren Alkalimetall-, Erdalkalimetall- oder Ammoniumsalze eingesetzt werden, beispielsweise Acrylsäure, Methacrylsäure, Dimethylacrylsäure, Ethylacrylsäure, Allylessigsäure, Vinylessigsäure und Vinylpropionsäure. Vorzugsweise verwendet man aus dieser Gruppe Acrylsäure, Methacrylsäure, deren Gemische sowie die Natrium-, Kalium-, Calcium- oder Ammoniumsalze oder deren Mischungen.

Als Comonomere (g) finden z.B. Alkyl, Hydroxyalkyl- und Vinylester wie Methylacrylat, Ethylacrylat, n-Butylacrylat, n-Butylacrylat, Methylmethacrylat, Hydroxyethylacrylat, Hydroxypropylacrylat, Hydroxybutylacrylat, Hydroxyethylmethacrylat, Hydroxypropylmethacrylat, Vinylformiat, Vinylacetat, Vinylpropionat sowie Mischungen derselben Verwendung. Die Anzahl der C-Atome in Alkyl- oder Hydroxyalkyl-Rest beträgt dabei vorzugsweise bis zu 18, insbesondere bis zu 12.

Die Comonomere (f) und (g) treten immer zusammen mit einem oder mehreren Monomeren (a) bis (e) auf und dienen hauptsächlich zur Modifizierung der erfindungsgemäßen Polymerisate. Die Comonomeren (f) und (g) werden üblicherweise in Mengen von 5 bis 95 Gew.-%, insbesondere 20 bis 80 Gew.-%, bezogen auf die Gesamtmenge aller Monomeren (A), eingesetzt.

Eine weitere Modifizierung der erfindungsgemäßen Polymerisate kann dadurch erreicht werden, daß die Monomeren oder Monomerenmischungen gegebenenfalls bis zu 5 Gew.-% eines mindestens zwei ethylenisch ungesättigte, nicht konjugierte Doppelbindungen im Molekül aufweisenden Comonomeren (h) enthalten. Werden die Verbindungen (h) mitverwendet, beträgt die bevorzugt verwendete Menge 0,05 bis 2 Gew.-%, jeweils bezogen auf die Gesamtmenge aller Monomeren (A).

Die Mitverwendung der Comonomeren (h) während der Polymerisation bewirkt eine Erhöhung der K-Werte der Copolymerisate. Geeignete Verbindungen dieser Art sind beispielsweise Ester von (Meth)acrylsäure mit mehrwertigen Alkoholen, z.B. Glykoldiacrylat, Glycerintriacrylat, Glyoldimethacrylat, Glycerintrimethacrylat, sowie mindestens zweifach mit Acrylsäure oder Methacrylsäure veresterte Polyole, wie Pentaerythrit und Glucose. Geeignete derartige Vernetzer sind außerdem Divinylbenzol, Divinyldioxan, Pentaerythrittriallylether und Pentaallylsucrose. Vorzugsweise verwendet man aus dieser Gruppe von Verbindungen (h) wasserlösliche Comonomere, wie Glykoldiacrylat oder Glykoldiacrylate von Polyethylenglykolen mit Molekulargewichten bis zu 3 000 g/mol.

In einer bevorzugten Ausführungsform werden als Monomere (A) entweder die Verbindungen (a) oder (b) jeweils alleine oder Mischungen aus 5 bis 95 Gew.-% einer Verbindung (b) und 95 bis 5 Gew.-% einer oder mehrerer der Monomere (a) eingesetzt.

Die Polymerisation der Monomeren (A) erfolgt in Gegenwart von Naturstoffen auf Basis von Monosacchariden, Oligosacchariden, Polysacchariden oder deren Derivaten (B). Diese Naturstoffe sind z.B. Saccharide pflanzlichen oder tierischen Ursprungs oder Produkte des Stoffwechsels von Mikroorganismen sowie deren Aufbau- und Modifizierungsprodukte, die bereits in Wasser, Alkalien oder Säuren dispergierbar oder löslich sind oder während der Polymerisation mit den Monomeren (A) direkt oder in teilweise oder völlig mit Säuren, Alkalien, Aminen oder Ammoniak neutralisierter Form dispergierbar oder löslich werden.

Es sind dies beispielsweise Pektin, Algin, Chitin, Chitosan, Heparin, Carrageenan, Agar, Gummiarabium, Tragant, Karaya-Gummi, Ghatti-Gummi, Johannisbrotkornmehl, Guar-Gummi, Tara-Gummi, Inulin, Xanthan, Dextran, Nigeran und Pentosane wie Xylan und Araban, deren Hauptbestandteile aus D-Glucuronsäure, D-Galakturonsäure, D-Galakturonsäuremethylester, D-Mannuronsäure, L-Guluronsäure, D- und L-Galaktose, 3,6-Anhydro-D-galaktose, Arabinose, L-Rhamnose, D-Xylose, L-Fucose, D-Mannose, D-Fructose und D-Glucose, 2-Amino-2-desoxy-d-glucose und 2-Amino-2-desoxy-D-galaktose sowie deren N-Acetylderivate bestehen.

Vom wirtschaftlichen Standpunkt aus gesehen verwendet man als Komponente (B) bei der Polymerisation vorzugsweise Stärken, thermisch und/ oder mechanisch behandelte Stärken, oxidativ, hydrolytisch oder enzymatisch abgebaute Stärken, oxidierte hydrolytisch oder oxidierte enzymatisch abgebaute Stärken, sowie chemisch modifizierte Stärken und chemisch modifizierte Monosaccharide und Oligosaccharide. Prinzipiell sind alle Stärken geeignet. Bevorzugt werden jedoch Stärken aus Mais, Weizen, Reis, Jopioku und insbesondere Stärke aus Kartoffeln. Die Stärken sind praktisch nicht in Wasser löslich und können in bekannter Weise durch thermische und/oder mechanische Behandlung oder durch einen enzymatischen oder einen säurekatalysierten Abbau in eine wasserlösliche Form übergeführt werden.

Als Stärkeabbauprodukte, die entweder durch oxidativen, hydrolytischen oder enzymatischen Abbau von Stärke erhältlich sind, seien beispielsweise folgende Verbindungen genannt: Dextrine, wie Weiß- und Gelbdextrine, Maltodextrine, Glucosesirup, Maltosesirup, Hydrolysate von hohem Gehalt an D-Glucose sowie Maltose und D-Glucose und deren Isomerisierungsprodukt Fructose. Als Komponente (B) eignen sich auch Mono- und Oligosaccharide, wie Galaktose, Mannose, Ribose, Saccharose, Raffinose, Laktose und Trehalose sowie Abbauprodukte der Cellulose, beispielsweise Cellubiose und ihre Oligomeren.

Farblose oder nur schwach gelb gefärbte wäßrige Lösungen der erfindungsgemäßen Polymerisate werden insbesondere dann erhalten, wenn man als Komponente (B) säurekatalytisch oder enzymatisch abgebaute Stärken einsetzt. Solche abgebauten Stärken sind im Handel unter der Bezeichnung Stärkeverzuckerungsprodukte erhältlich. Sie enthalten 0,5 bis 95, vorzugsweise 8 bis 20 Gew.-% Maltose, 2 bis 90 Gew.-% Maltotriose und höhere Zucker.

Als Komponente (B) kommen ebenso oxidierte Stärken, z.B. Dialdehydstärke und oxidierte Stärkeabbauprodukte in Betracht, beispielsweise Gluconsäure, Glucarsäure und Glucuronsäure. Solche Verbindungen werden beispielsweise durch Oxidation von Stärke mit Periodat, Chromsäure, Wasserstoffperoxid, Stickstoffdioxid, Stickstofftetroxid, Salpetersäure und Hypochlorit erhalten.

Als Komponente (B) eignen sich außerdem chemisch modifizierte Polysaccharide, insbesondere chemisch modifizierte Stärken, z.B. mit Säuren zu Estern und mit Alkoholen zu Ethern umgesetzte Stärken und Stärkeabbauprodukte. Die Veresterung dieser Stoffe ist sowohl mit anorganischen als auch mit organischen Säuren, deren Anhydriden oder Halogeniden möglich. Bei direkter Veresterung führt das freigesetzte Wasser zu einer säurekatalysierten Spaltung glykosidischer Bindungen. Von besonderem technischen Interesse sind phosphatierte und acetylierte Stärken und Stärkeabbauprodukte. Die gängigste Methode zur Veretherung von Stärke ist die Behandlung der Stärke und der Stärkeabbauprodukte mit organischen Halogenverbindungen, Epoxiden oder Sulfaten in wäßriger alkalischer Lösung. Stärkeether sind beispielsweise die Alkylether, Hydroxyalkylether, Carboxyalkylether und Allylether von Stärke. Unter chemisch modifizierten Stärken gemäß Komponente (B) sollen auch kationisch modifizierte Stärken verstanden werden, z.B. mit 2,3-Epoxipropyltrimethylammoniumchlorid umgesetzte Stärken.

Zu chemisch modifizierten Polysacchariden gehören beispielsweise auch Carboxymethylcellulose, Hydroxyethylcellulose, Hydroxypropylcellulose, Carboxymethylhydroxyethylcellulose, Sulfoethylcellulose, Carboxymethylsulfoethylcellulose, Hydroxypropylsulfoethylcellulose, Hydroxyethylsulfoethylcellulose, Methylsulfoethylcellulose und Ethylsulfoethylcellulose.

Als Komponente (B) eignen sich auch chemisch modifizierte abgebaute Stärken, beispielsweise Hydrierungsprodukte von Stärkehydrolysaten, wie Sorbit und Mannit, Maltit und hydrierter Glucosesirup oder oxidierte hydrolytisch abgebaute oder oxidierte enzymatisch abgebaute Stärken.

Ebenfalls geeignet sind die Produkte der sauer katalysierten oder enzymatischen Umglykosidierung oder Glykosidierung, wie z.B. Methylglukosid.

Zur Herstellung der erfindungsgemäßen Polymerisate werden die Monomeren (A) in Gegenwart der Verbindungen (B) radikalisch initiiert polymerisiert. In einigen Fällen kann es für die Wirkung des entstehenden Polymerisats günstig sein, zwei oder mehrere der unter (B) angegebenen Verbindungen einzusetzen, z.B. Mischungen aus einem Monosaccharid und einem Oligosaccharid, Mischungen aus einer enzymatisch abgebauten Stärke und einem Monosaccharid oder Mischungen aus Glucose und Saccharose oder Mannose.

Die Polymerisation kann in Gegenwart oder auch in Abwesenheit von inerten Lösemitteln oder inerten Verdünnungsmitteln durchgeführt werden. Da die Polymerisation in Abwesenheit von inerten Löse- oder Verdünnungsmitteln meistens zu uneinheitlichen Polymerisaten führt, wird die Polymerisation in einem inerten Löse- oder Verdünnungsmittel bevorzugt. Geeignet sind beispielsweise solche inerten Verdünnungsmittel, in denen die unter (B) angegebenen Verbindungen suspendiert werden können, und solche, die die Monomeren (A) lösen. In diesen Fällen liegen die Polymerisate nach der Reaktion in suspendierter Form vor und können leicht durch Filtration in fester Form isoliert werden. Geeignete inerte Verdünnungsmittel sind beispielsweise Toluol, o-, m-, p-Xylol und deren Isomerengemische, Ethylbenzol, aliphatische Kohlenwasserstoffe wie Pentan, Hexan, Heptan, Octan, Nonan, Dodecan, Cyclohexan, Cyclooctan, Methylcyclohexan sowie Mischungen der genannten Kohlenwasserstoffe oder Benzinfraktionen, die keine polymerisierbaren Monomeren enthalten. Außerdem eignen sich Chlorkohlenwasserstoffe wie Chloroform, Tetrachlorkohlenstoff, Hexachlorethan, Dichlorethan und Tetrachlorethan. Bei der beschriebenen Arbeitsweise, bei der die Ver(B) in einem inerten Verdünnungsmittel suspendiert sind, setzt man vorzugsweise wasserfreie Verbindungen (B) ein.

Eine bevorzugte Art der Herstellung der Polymerisate ist die Lösungspolymerisation, wobei die Verbindungen (B), die Monomeren (A) und das gebildete Polymerisat zumindest dispergiert, vorzugsweise in gelöster Form, vorliegen. Für die Lösungspolymerisation eignen sich beispielsweise inerte Lösungsmittel wie Methanol, Ethanol, Isopropanol, n-Propanol, n-Butanol, sek.-Butanol, Tetrahydrofuran, Dioxan sowie Mischungen der genannten inerten Lösemittel. Die Polymerisation kann kontinuierlich durchgeführt werden.

Die Komponente (A) und (B) können, wie bereits oben erwähnt, auch in Abwesenheit von inerten Verdünnungs- oder Lösemitteln polymerisiert werden. Hierfür bietet sich insbesondere die kontinuierliche Polymerisation bei Temperaturen von 160 bis 250°C an. In wenigen Fällen ist es hierbei möglich, in Abwesenheit von Polymerisationsinitiatoren zu arbeiten. Vorzugsweise setzt man jedoch auch hierbei solche Katalysatoren ein, die unter den Polymerisationsbedingungen Radikale bilden, z.B. anorganische und organische Peroxide, Persulfate, Azoverbindungen und sogenannte Redoxkatalysatoren.

Die erfindungsgemäßen Produkte, die durch Polymerisation der Komponente (A) und (B) erhalten werden, stellt man im allgemeinen unter Mitverwendung von radikalbildenden Initiatoren her. Als radikalbildende Initiatoren sind vorzugsweise alle diejenigen Verbindungen geeignet, die bei der jeweils gewählten Polymerisationstemperatur eine Halbwertszeit von weniger als 3 Stunden aufweisen. Falls man die Polymerisation zunächst bei niedriger Temperatur startet und bei höherer Temperatur zu Ende führt, ist es zweckmäßig, mit mindestens zwei bei verschiedenen Temperaturen zerfallenden Initiatoren zu arbeiten, nämlich zunächst einen bereits bei niedrigerer Temperatur zerfallenden Initiator für den Start der Polymerisation einzusetzen und dann die Hauptpolymerisation mit einem Initiator zu Ende zu führen, der bei höherer Temperatur zerfällt. Man kann wasserlösliche sowie wasserunlösliche Initiatoren oder Mischungen von wasserlöslichen und wasserunlöslichen Initiatoren einsetzen. Die in Wasser unlöslichen Initiatoren sind dann in der organischen Phase löslich.

Für die im folgenden angegebenen Temperaturbereiche kann man beispielsweise die dafür aufgeführten Initiatoren verwenden.

Temperatur: 40 bis 60°C

Acetylcyclohexansulfonylperoxid, Diacetylperoxidicarbonat, Dicyclohexylperoxidicarbonat, Di-2-ethylhexylperoxidicarbonat, tert.-Butylperneodecanoat, tert.-Amylperneodecanoat, 2,2'-Azobis-(4-methoxy-2,4-dimethylvaleronitril), 2,2'-Azobis-(2-amidinopropan)-dihydrochlorid, 2,2'-Azobis-[2-(2-imidazolin-2-yl)-propan]dihydrochlorid;

Temperatur: 60 bis 80°C

tert.-Butylperpivalat, tert.-Amylperpivalat, Dioctanoylperoxid, Dilaurylperoxid, 2,2'-Azobis-(2,4-dimethylvaleronitril);

Temperatur: 80 bis 100°C

Dibenzoylperoxid, tert.-Butylper-2-ethylhexanoat, tert.-Butylpermaleinat, 2,2'-Azobis-(isobutyronitril), Dimethyl-2,2'-azobisisobutyrat, Natriumpersulfat, Kaliumpersulfat, Ammoniumpersulfat;

Temperatur: 100 bis 120°C

Bis-(tert.-butylperoxi)-cyclohexan, tert.-Butylperoxiisopropyl-carbonat, tert.-Butylperacetat, Wasserstoffperoxid,

Temperatur: 120 bis 140°C

2,2-Bis-(tert.-butylperoxi)-butan, Dicumylperoxid, Di-tert.-amylperoxid, Di-tert.-butylperoxid;

Temperatur: > 140°C

p-Menthanhydroperoxid, Pinanhydroperoxid, Cumolhydroperoxid und tert.-Butylhydroperoxid.

Verwendet man zusätzlich zu den genannten Initiatoren noch Salze oder Komplexe von Schwermetallen, z.B. Kupfer-, Kobalt-, Mangan-, Eisen-, Vanadium-, Nickel- oder Chromsalze, oder organische Verbindungen, z.B. Benzoin, Dimethylanilin oder Ascorbinsäure, so können die Halbwertszeiten der angegebenen radikalbildenden Initiatoren verringert werden. So kann man beispielsweise tert.-Butylhydroperoxid unter Zusatz von 5 ppm Kupfer-III-acetylacetonat bereits so aktivieren, daß bereits bei 100°C polymerisiert werden kann.

Die reduzierende Komponente von Redoxkatalysatoren kann beispielsweise von Verbindungen wie Natriumsulfit, Natriumbisulfit, Natriumformaldehydsulfoxylat oder Hydrazin gebildet werden.

Bezogen auf die bei der Polymerisation eingesetzten Monomeren (A) verwendet man 0,01 bis 20, vorzugsweise 0,05 bis 10 Gew.-% eines Polymerisationsinitiators oder einer Mischung mehrerer Polymerisationsinitiatoren, als Redoxkomponenten setzt man 0,01 bis 15 Gew.-% der reduzierend wirkenden Verbindungen zu. Schwermetalle werden im Bereich von 0,1 bis 100 ppm, vorzugsweise 0,5 bis 10 ppm eingesetzt. Oft ist es von Vorteil, eine Kombination aus Peroxid, Reduktionsmittel und Schwermetall als Redoxkatalysator einzusetzen.

Die Polymerisation der Komponenten (A) und (B) kann auch durch Einwirkung von ultravioletter Strahlung, gegebenenfalls in Gegenwart von UV-Initiatoren, durchgeführt werden. Für das Polymerisieren unter Einwirkung von UV-Strahlen setzt man die dafür üblicherweise in Betracht kommenden Photoinitiatoren bzw. Sensibilisatoren ein. Hierbei handelt es sich beispielsweise um Verbindungen wie Benzoin oder Benzoinether, α-Methylbenzoin oder α-Phenylbenzoin. Auch sogenannte Triplett-Sensibilisatoren, wie Benzyldiketale, können verwendet werden. Als UV-Strahlungsquellen dienen beispielsweise neben energiereichen UV-Lampen, wie Kohlenbogenlampen, Quecksilberdampflampen oder Xenonlampen auch UV-arme Lichtquellen, wie Leuchtstoffröhren mit hohem Blauanteil.

Um Produkte mit niedrigem K-Wert herzustellen, wird die Polymerisation zweckmäßigerweise in Gegenwart von Reglern durchgeführt. Geeignete Regler sind beispielsweise Mercaptoverbindungen, wie Mercaptoethanol, Mercaptopropanol, Mercaptobutanol, Mercaptoessigsäure, Mercaptopropionsäure, Butylmercaptan und Dodecylmercaptan. Als Regler eignen sich außerdem Allylverbindungen, wie Allylalkohol, Aldehyde, wie Formaldehyd, Acetaldehyd, Propionaldehyd, n-Butyraldehyd und Isobutyraldehyd, Ameisensäure, Ammoniumformiat, Propionsäure, Hydrazinsulfat und Butenole. Falls die Polymerisation in Gegenwart von Reglern durchgeführt wird, benötigt man davon 0,05 bis 20 Gew.-%, bezogen auf die Gesamtmenge der bei der Polymerisation eingesetzten Monomeren (A).

Die Polymerisation erfolgt üblicherweise in einer Inertgasatmosphäre unter Ausschluß von Luftsauerstoff. Während der Polymerisation wird im allgemeinen für eine gute Durchmischung der Reaktionsteilnehmer gesorgt. Bei kleineren Ansätzen, bei denen eine sichere Abführung der Polymerisationswärme gewährleistet ist, kann man die Reaktionsteilnehmer, die vorzugsweise in einem inerten Verdünnungsmittel vorliegen, diskontinuierlich polymerisieren, indem man das Reaktionsgemisch auf die Polymerisationstemperatur erhitzt. Diese Temperaturen liegen in der Regel im Bereich von 40 bis 180°C.

Um den Verlauf der Polymerisationsreaktion besser kontrollieren zu können, gibt man die Monomeren (A) nach dem Anspringen der Polymerisation dem polymerisierenden Gemisch kontinuierlich oder absatzweise in dem Maße zu, daß die Polymerisation in Gegenwart der Komponenten (B) in dem gewünschten Temperaturbereich gut kontrollierbar ist. Bevorzugt ist eine Art der Zugabe der Monomeren (A), bei der man im Polymerisationsreaktor zunächst die Verbindungen (B) oder zumindest einen Teil der Verbindungen (B) zusammen mit mindestens einem Monomer (A) im Reaktor vorlegt und darin unter Rühren auf die gewünschte Polymerisationstemperatur erhitzt. Sobald diese Temperatur erreicht ist, fügt man über einen Zeitraum von etwa 1 bis 10, vorzugsweise 2 bis 8 Stunden, die Monomeren (A) sowie den Initiator und gegebenenfalls den Rest der Komponente (B) und falls erforderlich einen Regler zu. Eine derartige Vorgehensweise wird beispielsweise beim Polymerisieren der Komponenten (A) und (B) in einem inerten Verdünnungsmittel, in dem die Komponente (B) suspendiert ist, sowie auch bei der in Lösung durchgeführten Polymerisation angewendet.

Die erfindungsgemäßen Polymerisate werden durch Lösungspolymerisation der Komponenten (A) und (B) in wäßrigem Medium hergestellt, wobei die Lösungspolymerisation in Wasser besonders bevorzugt ist. Bei der Lösungspolymerisation in wäßrigem Medium geht man beispielsweise so vor, daß man einen Teil der Monomeren (A) und mindestens einen Teil der Verbindungen (B) in wäßrigemmedium vorlegt und den Rest der Monomeren (A) und gegebenenfalls den Rest der Verbindungen (B) kontinuierlich oder absatzweise dem polymerisierenden Reaktionsgemisch zufügt.

Wie bereits erwähnt, können die Monomeren (A) in wäßriger Systemen in Gegenwart von Polysacchariden polymerisiert werden. Vorzugsweise stellt man die Polymerisate, die aus Polysacchariden erhalten werden, dadurch her, daß man ein wasserunlösliches Polysaccharid zunächst in wäßriger Suspension unter Zusatz von Enzymen und/oder Säuren in eine wasserlösliche Form überführt und die dabei erhältliche wäßrige Lösung des abgebauten Polysaccharids mit Monomeren (A) vermischt, um diese in Gegenwart des abgebauten Polysaccharids zu polymerisieren. Hierbei wird zunächst ein wasserunlösliches Polysaccharid, wie beispielsweise Kartoffelstärke, in Wasser suspendiert und abgebaut. Dieser Abbau kann unter der Einwirkung von Enzymen, z.B. α- oder β-Amylose oder von entzweigenden Enzymen, wie z.B. Pullulanase, oder durch Einwirkung von organischen oder anorganischen Säuren in bekannter Weise vorgenommen werden. Als anorganische Säuren eignen sich beispielsweise Phosphorsäure, Schwefelsäure, Salzsäure und Salpetersäure. Geeignete organische Säuren sind beispielsweise gesättigte oder ungesättigte Carbonsäuren, z.B. Ameisensäure, Essigsäure, Propionsäure, Acrylsäure, Methacrylsäure, Maleinsäure, Itaconsäure, p-Toluolsulfonsäure und Benzolsulfonsäure.

Der enzymatische Abbau von Stärke wird üblicherweise im Temperaturbereich von 30 bis 120°C durchgeführt, während man den hydrolytischen Abbau der Stärke in der Regel bei Temperaturen von 50 bis 150°C vornimmt. Für den hydrolytischen Abbau benötigt man etwa 5 Minuten bis 10 Stunden, wobei der Grad des hydrolytischen Abbaus der Stärke von der gewählten Temperatur, dem pH-Wert und der Zeit abhängt. Nähere Angaben über den Abbau von Stärke können der Fachliteratur entnommen werden. In einigen Fällen hat es sich als vorteilhaft erwiesen, bereits beim enzymatischen oder hydrolytischen Abbau der Stärke mindestens eine der Phosphorverbindungen einzusetzen, die gemäß der Lehre der EP-A 175 317 zu Polymerisaten führen, die nicht oder nur sehr wenig gefärbt sind.

Bei der Polymerisation der Komponenten (A) und (B) liegen die Temperaturen üblicherweise im Bereich von 40 bis 180, vorzugsweise 50 bis 140 und besonders bevorzugt zwischen 60 bis 100°C. Sobald die Temperatur bei der Polymerisation oberhalb der Siedepunkte des inerten Verdünnungs- oder Lösungsmittel oder der Monomeren (A) liegt, wird die Polymerisation unter Druck durchgeführt. Die Konzentration der Komponenten (A) und (B) beträgt bei der Polymerisation in Gegenwart von inerten Löse- oder inerten Verdünnungsmitteln 10 bis 80, vorzugsweise 20 bis 70 Gew.-%.

Die Herstellung der erfindungsgemäßen Polymerisate kann in üblichen Polymerisationsvorrichtungen erfolgen. Hierzu verwendet man beispielsweise Rührkessel, die mit einem Anker-, Blatt-, Impeller- oder Mehrstufenimpulsgegenstromrührer ausgestattet sind. Insbesondere bei Abwesenheit von Verdünnungsmitteln kann es vorteilhaft sein, die Polymerisation in Knetern durchzuführen. Ebenso kann es notwendig sein, in einem Kneter zu polymerisieren, wenn man bei hohen Konzentrationen arbeitet oder wenn die Naturstoffe (B) hochmolekular sind und zunächst stark quellen.

Man erhält Polymerisate, die, soweit sie in Wasser löslich sind, K-Werte von 8 bis 350, vorzugsweise 10 bis 250, haben (gemessen an 1 gew.-%igen wäßrigen Lösungen der Polymerisate bei pH 7 und 25°C). Nimmt man die Messungen an 0,1 gew.-%igen wäßrigen Lösungen bei pH 7 und 25°C vor, liegen die K-Werte im Bereich von 8 bis 450, vorzugsweise 10 bis 350. Die nach den oben angegebenen Verfahren herstellbaren Produkte sind farblos bis gelblich. Sie liegen beim Polymerisieren in wäßrigem Medium als Dispersionen oder Polymerlösungen vor. In Abhängigkeit von der jeweiligen Zusammensetzung der Polymerisate handelt es sich dabei um niedrigviskose bis pastöse wäßrige Lösungen oder wäßrige Dispersionen.

Um die Eigenschaften der erfindungsgemäßen Polymerisate zu verbessern, kann es in manchen Fällen vorteilhaft sein, die erhaltenen Produkte nachträglich einer oxidativen Behandlung zu unterziehen. Zu diesem Zweck läßt man Oxidationsmittel entweder auf die pulverförmigen Polymerisate direkt einwirken oder auf Suspensionen der erhaltenen Produkte in einem inerten Suspensionsmittel oder auch auf Lösungen der erhaltenen Produkte in einem inerten Lösemittel, z.B. in einem einwertigen Alkohol wie Methanol, Ethanol, n-Propanol oder Isopropanol oder vorzugsweise Wasser oder in Mischungen aus Wasser und Alkoholen. Die Oxidation wird vorzugsweise in wäßrigen Lösungen der erhaltenen Produkte durchgeführt.

Als Oxidationsmittel kommen solche in Frage, die beim Erhitzen allein oder in Gegenwart von Katalysatoren Sauerstoff freisetzen. Geeignete organische Verbindungen sind allgemein Peroxide, die sehr leicht aktiven Sauerstoff abspalten. Bei niedrigen Temperaturen haben nur Hydroperoxide und Persäuren eine deutliche oxidierende Wirkung; Perester, Diacylperoxide und Dialkylperoxide wirken erst bei höheren Temperaturen. Geeignete Peroxide sind beispielsweise Diacetylperoxid, Isopropylpercarbonat, tert.-Butylhydroperoxid, Cumolhydroperoxid, Acetylacetonperoxid, Methylethylketonperoxid, Di-tert.-butylperoxid, Dilaurylperoxid, tert.-Butylperpivalat, tert.-Amylperpivalat, tert.-Butylperethylhexanoat, tert.-Amylperethylhexanoat, tert.-Butylperneodecanoat und tert.-Amylperneodecanoat.

Bevorzugt sind die preisgünstigen anorganischen Oxidationsmittel, die sich besonders gut für die Oxidation von wäßrigen Lösungen der carbonylgruppenhaltigen Polymeren eignen. Beispielsweise seien genannt Chlor, Brom, Iod, Salpetersäure, Natriumpermanganat, Kaliumchlorat, Natriumhypochlorit, Natriumperborat, Natriumpercarbonat und Natriumpersulfat. Ein besonders bevorzugtes Oxidationsmittel ist Wasserstoffperoxid.

Der Zerfall der Perverbindungen, also die Oxidation, kann durch den Zusatz von Beschleunigern oder Aktivatoren forciert werden. Diese Beschleuniger oder Aktivatoren sind reduzierend wirkende Stoffe wie beispielsweise tert.-Amine, Sulfinsäuren, Dithionite, Sulfite, α- und β-Ketocarbonsäuren, Glukosederivate sowie Schwermetalle, vorzugsweise in Form löslicher Salze anorganischer oder organischer Säuren oder Komplexe. Namentlich seien genannt Dimethylanilin, Dimethyl-p-toluidin, Diethylanilin, Natriumdithionit, Natriumsulfit, Ascorbinsäure, Glukose, Pentaacetylglykose, Eisen-II-ammoniumsulfat, Kupfer-I-chlorid und die Acetylacetonate von Eisen, Kupfer, Kobalt, Chrom, Mangan, Nikkel und Vanadium.

Die Oxidationsmittel werden, berechnet auf die Summe der Komponenten (A) und (B), vorteilhafterweise in Mengen von 1 bis 50 Gew.-%, vorzugsweise 5 bis 30 Gew.-%, zugesetzt. Die Reduktionsmittel werden, berechnet auf die Oxidationsmittel, in Mengen von 2 bis 50 Gew.-% angewendet. Die Schwermetallverbindungen setzt man, berechnet als Schwermetall und bezogen auf die Polymeren aus (A) und (B), in Mengen von 0,1 bis 100 ppm, vorzugsweise 0,5 bis 10 ppm ein. Oftmals ist es von Vorteil zur Beschleunigung der Reaktion, besonders wenn bei niedrigen Temperaturen gearbeitet wird, den Perverbindungen sowohl Reduktionsmittel als auch Schwermetallverbindungen zuzusetzen.

Die Reaktionstemperaturen für die Oxidation können sich zwischen 20 und 150°C, bevorzugt 50 bis 120°C, bewegen. Manchmal ist es auch vorteilhaft, die Oxidation durch Bestrahlen mit UV-Licht zu beschleunigen, oder auch bei niedrigen Temperaturen und kurzer Zeit zu oxidieren. Dadurch bewirkt man, daß der K-Wert der erfindungsgemäßen Polymerisats nicht merklich erniedrigt wird. Auch Luft und Sauerstoff können allein oder in Kombination mit Oxidationsmittel zur Oxidation der erfindungsgemäß erhaltenen Produkte Verwendung finden.

Die erfindungsgemäß hergestellten Polymerisate sind bevorzugt substantive, kationische Verbindungen, die als filmbildende Konditioniermittel mit verbesserter Wirksamkeit in kosmetischen Zubereitungen, insbesondere in Haar- und Hautpflegemitteln, eingesetzt werden können.

Sie sind in kosmetischen Zubereitungen in einer Menge von 0,1 bis 35 Gew.-%, insbesondere 1 bis 20 Gew.-%, als filmbildende Konditioniermittel enthalten.

Auf dem Gebiet der Haarkosmetik verbessern diese Produkte als Bestandteile in Conditionern, Shampoos, Haarfestigern, Haarspül-, Well-, Bleich- und Färbemitteln die Naßkämmbarkeit der Haare und verhindern die elektrostatische Aufladung des trockenen Haars. Bei geschädigtem Haar wird die Haaroberfläche geglättet und die Festigkeit erhöht (Kurwirkung). Durch ihre filmbildenden Eigenschaften verleihen die beschriebenen Produkte den Haaren mehr Volumen.

Dank der sehr guten Verträglichkeit mit anionenaktiven Tensiden und anionischen Polymeren eignen sich die erfindungsgemäß hergestellten Polymerisate besonders als Wirksubstanz in Conditioner-Shampoos, Badegelen und ähnlichen Zubereitungen. Erfindungsgemäßene Polymerisate mit geringer Kationenaktivität sind verträglich mit Carbomer-Polymeren und eignen sich zur Herstellung von Conditioner-Gelen.

Für Conditioner und Haarfestiger benötigt man bevorzugt Produkte mit K-Werten von 100 bis 350 bzw. 150 bis 450 (gemessen an 1 gew.-%igen bzw. 0,1 gew.-%igen wäßrigen Lösungen der Polymerisate bei pH 7 und 25°C), während man für Shampoos, Haarspül-, Bleich- und Färbemittel Produkte mit K-Werten von 8 bis 200 bzw. 8 bis 250 (gemessen an 1 gew.-%igen bzw. 0,1 gew.-%igen wäßrigen Lösungen der Polymerisate bei pH 7 und 25°C) benötigt.

Als Zusätze zu Hautpflegepräparaten verbessern diese Produkte deren Spreitung und vermitteln ein glattes und geschmeidiges Hautgefühl. Die Qualität von Rasierpräparaten und Seifen wird durch die Schaumstabilisierung bzw. die Gleitwirkung der eingesetzten Produkte verbessert. Sie vermitteln ein seidiges Hautgefühl, das auch nach dem Abspülen erhalten bleibt.

Erfindungsgemäß hergestellte Polymerisate, hergestellt mit überwiegendem Anteil an Monomeren b), eignen sich insbesondere als polymere Bestandteile in Iodophoren (Iodkomplexe für medizinische Anwendungen), als Klebstoffe, z.B. in Klebstiften oder als Farbübertragungsverhinderer, z.B. in Waschmitteln oder in der Textilindustrie. Als Mittel zur Verhinderung der Farbübertragung eignen sich auch erfindungsgemäß hergestellte Polymere, hergestellt mit überwiegendem Anteil an Monomeren (a) oder Gemischen aus Monomeren a) und b).

Mit erfindungsgemäß gergestellten Polymerisaten hergestellte Iodophore weisen eine verbesserte Lagerstabilität auf. Bei der Verwendung als Farbübertragungsinhibitoren ergibt sich gegenüber dem Stand der Technik ebenfalls eine Verbesserung.

Weiterhin können die erfindungsgemäß hergestellten Polymerisate als Stabilisatoren für Parfüme und Parfümöle verwendet werden.

In Parfümen und Parfümölen sind sie in einer Menge von 0,1 bis 15 Gew.-%, insbesondere 1 bis 10 Gew.-%, als Stabilisatoren enthalten.

Die erfindungsgemäß hergestellten Polymerisate eignen sich auch als Leitfähigkeitsharze, als Flockungsmittel und als Hilfsmittel bei der Ölgewinnung.

Ein weiterer Vorteil der erfindungsgemäß hergestellten Polymerisate ist ihre weitgehende biologische Abbaubarkeit bzw. Eliminierbarkeit aus dem Abwasser von Kläranlagen. Nicht zu vergessen ist der Aspekt des Rückgriffs bei den beschriebenen Produkten auf nachwachsende Rohstoffe zur weiteren Verbesserung der ökologischen Akzeptanz bei vergleichbarem oder sogar verbessertem Eigenschaftsprofil zu rein synthetischen Kosmetikprodukten. Die erfindungsgemäß hergestellten Polymerisate mit überwiegendem Anteil an Monomeren (b) eignen sich des weiteren für alle Anwendungsgebiete, in denen PVP Verwendung findet.

### Beispiele

Falls nicht anders bezeichnet, bedeuten die Angaben in Prozent Gewichtsprozent.

### Herstellung und Eigenschaften der Polymerisate

Die K-Werte der Polymerisate wurden nach H. Fikentscher, Cellulosechemie, Band 13, S. 58 bis 64 und S. 71 bis 74 (1932), bestimmt, dabei bedeutet K = k x 10³. Die Messungen wurden an 1 %igen bzw. 0,1 %igen wäßrigen Lösungen der Polymerisate bei 25°C und einem pH-Wert von 7 durchgeführt.

Die DE-Werte der Stärken oder ihrer Derivate bedeuten Dextrose-Äquivalente. Sie wurden nach der Methode von Luff-Schoorl bestimmt (s.G. Tegge, Stärke und Stärkederivate, Behr's Verlag, Hamburg 1984, Seite 305).

### Beispiel 1

Eine Mischung aus 180 g mit Dimethylsulfat quaternisiertem Vinylimidazol, 80 g Vinylpyrrolidon und 460 ml Wasser bildete den Zulauf 1. Aus Zulauf 1, 44 g 2,2'-Azobis(2-amidinopropan)dihydrochlorid und 100 ml Wasser wurde Zulauf 2 hergestellt. In einem 2 l-Rührkolben, der mit Rührer, Heizung, Rückflußkühler, Thermometer, Dosiervorrichtungen, Stickstoffein- und auslaßvorrichtungen ausgestattet war, wurden 80 g Saccharose, 300 ml Wasser, 200 ml Zulauf 1 und 35 ml Zulauf 2 unter Rühren auf 60°C erhitzt. Nach dem Erreichen der vorgesehenen Temperatur wurden der restliche Zulauf 1 innerhalb von 5 Stunden und der restliche Zulauf 2 innerhalb von 6 Stunden bei gleichbleibender Temperatur von 60°C zudosiert. Anschließend wurde noch 2 Stunden lang bei dieser Temperatur gerührt. Man erhielt eine klare, hochviskose Polymerlösung mit einem Feststoffgehalt von 20,4 %. Der K-wert (0,1 %ig in Wasser) betrug 238,5.

### Beispiel 2

Beispiel 1 wurde mit der Abänderung wiederholt, daß man anstelle von Saccharose die gleiche Mange Maltodextrin mit einem DE-Wert von 11 bis 14 einsetzte. Man erhielt eine klare, farblose, hochviskose Lösung mit einem Feststoffgehalt von 20,2 %. Der K-Wert (0,1 %ig in Wasser) betrug 257,5. Der Restvinylpyrrolidongehalt lag unter der Nachweisgrenze (ca. 10 ppm).

### Beispiel 3

Beispiel 1 wurde mit der Abänderung wiederholt, daß man anstelle von 2,2'-Azobis(2-amidinopropan)dihydrochlorid 1,45 g Kaliumperoxidisulfat eingesetzte und bei 90°C polymerisierte. Man erhielt eine klare, viskose Lösung mit einem Feststoffgehalt von 19,8 %. Der K-Wert (0,1 %ig in Wasser) betrug 162,3.

### Beispiel 4

Beispiel 2 wurde mit der Abänderung wiederholt, daß man anstelle von 2,2'-Azobis(2-amidinopropan)dihydrochlorid als Zulauf 2 eine Mischung aus 1,2 g H₂O₂ (30 %ig in Wasser) 0,05 g FeCl₃ x 6 H₂O und 100 ml Wasser einsetzte und bei 80°C polymerisierte. Man erhielt eine klare, viskose Lösung mit einem Feststoffgehalt von 20,1 %. Der K-Wert (0,1 %ig in Wasser) betrug 168,4.

### Beispiel 5

Zulauf 1 bestand aus einer Mischung von 515 g mit Methylchlorid quaternisiertem Vinylimidazol, 600 ml Wasser und 50 g Isopropanol. Aus 4,6 g tert.-Butyl-2-ethylhexanoat und 157 g Isopropanol wurde Zulauf 2 hergestellt. In einem 2 1-Rührkolben, der mit Heizung, Rührer, Rückflußkühler, Stickstoffein- und -auslaß und Dosiervorrichtungen ausgestattet war, wurden 180 g Kartoffelstärke, 180 ml Wasser, 120 ml Zulauf 1 und 20 ml Zulauf 2 vorgelegt und bis zum schwachen Sieden unter Rückfluß erhitzt (ca. 85°C). Dann wurden unter ständigem schwachen Sieden der restliche Zulauf 1 innerhalb von 6 Stunden, der restliche Zulauf 2 innerhalb von 8 Stunden zudosiert und anschließend noch eine Stunde lang am Sieden gehalten. Anschließend wurde das Isopropanol durch Einleiten von Wasserdampf ausgetrieben. Man erhielt eine klare, viskose Polymerlösung. Der K-Wert des Polymeren betrug 55,2 (1 %ig in Wasser).

### Beispiel 6

Zulauf 1 bestand aus einer Mischung von 203 g mit Dimethylsulfat quaternisiertem Vinylimidazol, 203 g Vinylpyrrolidon und 220 g Wasser. Als Zulauf 2 diente eine Lösung von 1,4 g 2,2'-Azobis(2-amidinopropan)hydrochlorid in 75 g Wasser. In einem 2 l-Glasgefäß mit Rührer, Heizung, Rückflußkühler, Stickstoffein- und -auslaß und Dosiervorrichtungen wurden 200 g Wasser, 106 ml Zulauf 1, 5 ml Zulauf 2 und 100 g Maltodextrin mit einem DE-Wert von 11 bis 14 vorgelegt und auf 65°C erwärmt. Dann wurden bei dieser Temperatur der restliche Zulauf 1 in 5 Stunden und der restliche Zulauf 2 in 7 Stunden zudosiert und noch eine Stunde nachgerührt. Man erhielt eine klare, viskose Polymerlö-sung. Der K-Wert der Polymeren betrug 125,4 (1 %ig in Wasser). Der Restvinylpyrrolidongehalt lag unter der Nachweisgrenze (< 10 ppm) und der Feststoffgehalt nach Verdünnen mit 800 ml Wasser bei 28,0 %.

### Beispiel 7

Beispiel 6 wurde mit der Abänderung wiederholt, daß man anstelle von Maltodextrin die gleiche Menge Dextrin (weiß) verwendete. Der Feststoffgehalt lag bei 48,6 % und der K-Wert (1 %ig in Wasser) bei 124,1.

### Beispiel 8

Zulauf 1 bestand aus einer Mischung von 203 g mit Methylchlorid quaternisiertem Vinylimidazol, 200 g Vinylpyrrolidon, 240 g Wasser und 1 g 2-Merkaptoethanol. Als Zulauf 2 diente eine Lösung von 2,4 g 2,2'-Azobis-(2-amidinopropan)dihydrochlorid in 75 g Wasser. In einem 2 l-Glasgefäß, das mit Rührer, Heizung, Rückflußkühler, Gasein- und -auslaß und Dosiervorrichtungen ausgestattet war, wurden 200 g Wasser, 100 ml Zulauf 1, 10 ml Zulauf 2 und 100 g Kartoffelstärke (82,4 %ig) vorgelegt und auf 60°C erwärmt. Dann wurden bei dieser Temperatur der restliche Zulauf 1 in 5 Stunden und der restliche Zulauf in 7 Stunden zudosiert und noch eine Stunde nachgerührt. Man erhielt eine klare, viskose Polymerlösung mit einem Feststoffgehalt von 48,3 %. Der K-Wert (1 %ig in Wasser) betrug 76,3.

### Beispiel 9

Beispiel 8 wurde mit der Abänderung wiederholt, daß man anstelle von Kartoffelstärke die gleiche Menge Glukose verwendete. Man erhielt eine klare, viskose Polymerlösung mit einem Feststoffgehalt von 49,9 % und einem K-Wert (1 %ig in Wasser) von 72,1.

### Beispiel 10

Zulauf 1 bestand aus einer Mischung von 153 g mit Methylchlorid quaternisiertem Vinylimidazol, 150 g Vinylpyrrolidon, 150 g Glukose und 350 g Wasser. Als Zulauf 2 diente eine Lösung von 2,0 g tert.-Butylperneodecanoat in 30 g Ethanol. In einem 2 l-Gefäß, das mit Rührer, Heizung, Rückflußkühler, Gasein- und -auslaß und Dosiervorrichtungen ausgestattet war, wurden 400 g Wasser, 200 ml Zulauf 1 und 10 ml Zulauf 2 vorgelegt und auf 55°C erwärmt. Dann wurden bei dieser Temperatur der restliche Zulauf 1 in 5 Stunden und der restliche Zulauf 2 in 7 Stunden zudosiert und noch eine Stunde nachgerührt. Man erhielt eine klare, viskose Polymerlösung mit einem Feststoffgehalt von 36,1 %. Der K-Wert lag bei 118,3 (1 %ig in Wasser).

### Beispiel 11

Beispiel 10 wurde mit der Abänderung wiederholt, daß man anstelle von tert.-Butylperneodecanoat die gleiche Menge Di-tert.-butylperoxid verwendete. Man polymerisierte bei 2,2 bar und 125°C in einem 2 1-Druckbehälter und erhielt eine gelbe, klare, viskose Lösung mit einem Feststoffgehalt von 37,4 %. Der K-Wert lag bei 92,4 (1 %ig in Wasser).

### Beispiel 12

Zulauf 1 bestand aus einer Mischung von 120,0 g mit Methylchlorid quaternisiertem Vinylimidazol und 400 ml Wasser. Als Zulauf 2 diente eine Lösung von 1,84 g 2,2'-Azobis-(2-amidinopropan)dihydrochlorid und 100 ml Wasser. In einem 2 l-Rührkolben, der mit Rührer, Heizung, Rückflußkühler, Gasein- und -auslaß und Dosiervorrichtungen ausgestattet war, wurden 120 g Saccharose, 300 ml Wasser, 200 ml Zulauf 1 und 35 ml Zulauf 2 vorgelegt und es wurde auf 65°C erwärmt. Dann wurden bei dieser Temperatur der restliche Zulauf 1 in 5 Stunden und der restliche Zulauf 2 in 6 Stunden zudosiert und noch drei Stunden nachgerührt. Man erhielt eine gelbe, klare, viskose Polymerlösung mit einem Feststoffgehalt von 20,2 %. Der K-Wert der erhaltenen Lösung lag bei 88,7 % (1 %ig in Wasser).

### Beispiel 13

Zulauf 1 bestand aus einer Mischung von 80 g mit Dimethylsulfat quaternisiertem Vinylimidazol, 80 g Vinylpyrrolidon und 560 g Wasser. Als Zulauf 2 diente eine Lösung von 1,44 g 2,2'-Azobis(2-amidinopropan)dihydrochlorid und 100 ml Wasser. In einem 2 l-Rührkolben, der mit Rührer, Heizung, Rückflußkühler, Gasein- und -auslaß und Dosiervorrichtung ausgestattet war, wurden 80 g Saccharose, 250 ml Wasser, 200 ml Zulauf 1 und 35 ml Zulauf 2 vorgelegt und auf 60°C erhitzt. Bei dieser Temperatur wurden der restliche Zulauf 1 in 5 Stunden und der restliche Zulauf 2 in 6 Stunden zudosiert und noch zwei Stunden nachgerührt. Man erhielt eine klare, viskose Polymerlösung mit einem Feststoffgehalt von 20,6 %. Der K-Wert der erhaltenen Lösung lag bei 186,0 (0,1 %ig in Wasser).

### Beispiel 14

Zulauf 1 bestand aus einer Mischung, die der aus Beispiel 13 entsprach. Als Zulauf 2 diente ebenfalls eine Lösung von 1,44 g 2,2'-Azobis(2-amidinopropan)dihydrochlorid in 100 ml Wasser. In einem 2 l-Rührkolben, der mit Rührer, Heizung, Rückflußkühler, Gasein- und -auslaß und Dosiervorrichtung ausgestattet war, wurden 80 g Saccharose, 200 ml Wasser, 200 ml Zulauf 1 und 35 ml Zulauf 2 vorgelegt und auf 60°C erhitzt. Bei dieser Temperatur wurden der restliche Zulauf 1 in 5 Stunden und der restliche Zulauf 2 in 6 Stunden zudosiert. Nach Zugabe von 100 ml Wasser erhielt man eine klare, farblose, hochviskose Polymerlösung mit einem Feststoffgehalt von 19,6 %. Der K-Wert der erhaltenen Lösung lag bei 262,9 (0,1 %ig in Wasser).

### Beispiel 15

Zulauf 1 bestand aus einer Mischung von 133 g Vinylpyrrolidon, 51,5 g mit Diethylsulfat quaternisiertem Dimethylaminopropylmethacrylamid und 325 g Wasser. Zulauf 2 bestand aus 0,44 g 2,2'-Azobis-(2-amidinopropan)dihydrochlorid und 25 g Wasser. In einem 2 l-Glaskolben, der mit Rührer, Heizung, Rückflußkühler, Gasein- und auslaß und Dosiervorrichtung versehen war, wurden 80 g Saccharose, 390 ml Wasser, 165 ml Zulauf 1 und 8 ml Zulauf 2 vorgelegt und auf 65°C erhitzt. Bei dieser Temperatur wurden der restliche Zulauf 1 in 4 Stunden und der restliche Zulauf 2 in 6 Stunden zudosiert und noch zwei Stunden nachgerührt. Man erhielt eine klare, viskose Polymerlösung mit einem Feststoffgehalt von 25,0 %. Der K-Wert der erhaltenen Lösung betrug 223,3 (0,1 %ig in Wasser).

### Beispiel 16

Beispiel 15 wurde mit der Abänderung wiederholt, daß man anstelle von Saccharose die gleiche Menge Maltodextrin mit einem DE-Wert (nach Luff-schoorl) von 11 bis 14 einsetzte. Man erhielt eine fast klare, hochviskose Polymerlösung mit einem Feststoffgehalt von 25,6 %. Der K-Wert der erhaltenen Lösung lag bei 119,3 (1 %ig in Wasser).

### Beispiel 17

Zulauf 1 bestand aus einer Mischung von 180 g Vinylimidazol und 325 g Wasser. Als Zulauf 2 diente eine Lösung von 1,0 g 2,2'-Azobis-(2-amidinopropan)dihydrochlorid in 100 ml Wasser. In einem 2 l-Rührkolben mit Rückflußkühler, Rührer, Thermometer, Heizung, Gasein- und -auslaß und Dosiervorrichtung wurden 90 g Saccharose, 390 ml Wasser, 170 ml Zulauf 1 und 35 ml Zulauf 2 vorgelegt und auf 60°C erhitzt. Bei dieser Temperatur wurden der restliche Zulauf 1 in 5 Stunden und der restliche Zulauf 2 in 7 Stunden zudosiert und 4 Stunden bei 60°C nachgerührt. Man erhielt eine gelbe, klare, viskose Lösung mit einem Feststoffgehalt von 24,9 %. Der K-Wert der erhaltenen Lösung lag bei 83,4 (1 %ig in Wasser).

### Beispiel 18

Beispiel 17 wurde mit der Abänderung wiederholt, daß man anstelle von Saccharose enzymatisch abgebaute Kartoffelstärke mit einem DE-Wert von 37,5 bis 40,5 (nach Luff-Schoorl) verwendete. Man erhielt eine klare, gelbe, viskose Polymerlösung mit einem Feststoffgehalt von 25,1 %. Der K-Wert der erhaltenen Lösung lag bei 79,5 (1 %ig in Wasser).

### Beispiel 19

Zulauf 1 bestand aus einer Mischung von 80 g Dimethylaminoethylmethacrylat, 80 g Vinylpyrrolidon und 560 g Wasser. Als Zulauf 2 diente eine Lösung aus 1,44 g 2,2'-Azobis-(2-amidinopropan)dihydrochlorid in 100 ml Wasser. In einem 2 l-Glasgefäß, das mit Rückflußkühler, Rührer, Thermometer, Heizung, Gasein- und -auslaß und Dosiervorrichtungen ausgestattet war, wurden 80 g Saccharose, 300 ml Wasser, 200 ml Zulauf 1 und 35 ml Zulauf 2 vorgelegt und auf 60°C erhitzt. Bei dieser Temperatur wurden der restliche Zulauf 1 in 5 Stunden und der restliche Zulauf 2 in 6 Stunden zudosiert und 2 Stunden bei 60°C nachgerührt. Man erhielt ein klares, farbloses, teilweise vernetztes Polymergel mit einem Feststoffgehalt von 20,3 %. Der K-Wert oder die relative Viskosität konnten wegen der Vernetzung nicht bestimmt werden.

### Beispiel 20

Zulauf 1 bestand aus einer Mischung von 60 g Dimethylaminopropylmethacrylamid, 60 g Vinylpyrrolidon und 560 g Wasser. Als Zulauf 2 diente eine Lösung aus 1,44 g 2,2'-Azobis-(2-amidinopropan)dihydrochlorid in 100 ml Wasser. In einem 2 l-Rührkolben mit Rückflusskühler, Rührer, Thermometer, Heizung, Gasein- und -auslass und Dosiervorrichtungen wurden 120 g Saccharose, 300 ml Wasser, 200 ml Zulauf 1 und 35 ml Zulauf 2 vorgelegt und auf 60<0>C erhitzt. Bei dieser Temperatur wurden der restliche Zulauf 1 in 5 Stunden und der restliche Zulauf 2 in 6 Stunden zudosiert und 2 Stunden bei 60<0>C nachgerührt. Man erhielt eine hochviskose Polymerlösung mit einem Festgehalt von 21,5 % und einem K-Wert von 280,6 (0,1 %ig in Wasser).

### Beispiel 21

Zulauf 1 bestand aus einer Mischung von 80 g mit Methylchlorid quaternisiertem Vinylimidazol, 80 g Vinylpyrrolidon und 560 ml Wasser. Als Zulauf 2 diente eine Mischung aus 1,44 g 2,2'-Azobis-[2-(2-imidazolin-2-yl)propan]dihydrochlorid in 100 ml Wasser. In einem 2 l-Rührkolben mit Rückflußkühler, Rührer, Thermometer, Heizung, Gasein- und -auslaß und Dosiervorrichtungen wurden 80 g Hydroxypropylcellulose, 300 ml Wasser, 200 ml Zulauf 1 und 35 ml Zulauf 2 vorgelegt und auf 45°C erhitzt. Bei dieser Temperatur wurden der restliche Zulauf 1 in 5 Stunden und der restliche Zulauf 2 in 6 Stunden zudosiert und 2 Stunden bei 60°C nachgerührt. Man erhielt eine leicht trübe, viskose Polymerlösung mit einem Feststoffgehalt von 21,4 %. Der K-Wert der erhaltenen Lösung betrug 204,0 (0,1 %ig in Wasser).

### Beispiel 22

Zulauf 1 bestand aus einer Mischung von 80 g mit Methylchlorid quaternisiertem Vinylimidazol, 80 g Vinylpyrrolidon und 560 ml Wasser. Als Zulauf 2 diente eine Mischung aus 1,4 g 2,2'-Azobis-(2-amidinopropan)dihydrochlorid in 100 ml Wasser. In einem 2 l-Rührkolben mit Rückflußkühler, Rührer, Heizung, Gasein- und -auslaß und Dosiervorrichtungen wurden 20 g Natrium-Carboxymethylcellulose, 300 g Wasser, 200 ml Zulauf 1 und 35 ml Zulauf 2 vorgelegt und auf 65°C erhitzt. Bei dieser Temperatur wurden der restliche Zulauf 1 in 4 Stunden und der restliche Zulauf 2 in 6 Stunden zudosiert und 2 Stunden nachgerührt. Man erhielt eine leicht trübe, viskose Polymerlösung mit einem Feststoffgehalt von 20,3 %. Der K-Wert der erhaltenen Lösung lag bei 199,8 (0,1 %ig in Wasser).

### Beispiel 23

Zulauf 1 bestand aus einer Mischung von 96 g Methylchlorid quaternisiertem Vinylimidazol, 384 g Vinylpyrrolidon, 216 ml Wasser und 0,96 g Merkaptoethanol. Als Zulauf 2 diente eine Mischung aus 1,68 g 2,2'-Azobis-(2-amidinopropan)dihydrochlorid in 78 ml Wasser. In einem 2 l-Rührkolben mit Rückflußkühler, Rührer, Thermometer, Heizung, Gasein- und -auslaß, wurden 30 g Maltodextrin mit einem DE-Wert von 11 bis 14 (nach Luff-Schoorl), 60 ml Zulauf 1, 6 ml Zulauf 2 und 420 ml Wasser vorgelegt und auf 65°C erhitzt. Bei dieser Temperatur wurden der restliche Zulauf 1 in 4 Stunden und der restliche Zulauf 2 in 6 Stunden zudosiert und 2 Stunden nachgerührt. Man erhielt eine klare, viskose Polymerlösung mit einem Feststoffgehalt von 44,6 %. Der K-Wert der erhaltenen Lösung betrug 69,4 (0,1 %ig in Wasser).

### Beispiei 24

Zulauf 1 bestand aus einer Mischung von 200 g Dimethyldiallylammoniumchlorid und 300 g Wasser. Als Zulauf 2 diente 2,9 g 2,2'-Azobis-(2-amidinopropan)dihydrochlorid in 100 ml Wasser. In einem 2 l-Glasgefäß, das mit Rückflußkühler, Rührer, Thermometer, Beizung Gasein- und -auslaß bestückt war, wurden 100 g Kartoffel stärke 150 ml Zulauf 1, 35 ml Zulauf 2 und 200 ml Wasser vorgelegt und auf 65°C erhitzt. Bei dieser Temperatur wurden der restliche Zulauf 1 in 6 Stunden und der restliche Zulauf 2 in 8 Stunden zudosiert und 2 Stunden nachgerührt. Man erhielt eine gelbe, viskose Lösung mit einem Feststoffgehalt von 27,9 %. Der K-wert der erhaltenen Polymerlösung betrug 47,9 (0,1 %ig in Wasser).

### Vergleichsbeispiele 25 bis 54

### Beispiel 25

Dieses Beispiel entspricht dem Beispiel 13 aus der Literaturstelle (1). Man erhielt eine klare, viskose Polymerlösung mit einem Feststoffgehalt von 39,8 % (0,1 %ig in Wasser).

### Beispiel 26

Zulauf 1 bestand aus einer Mischung von 203 g mit Methylchlorid quaternisiertem Vinylimidazol, 200 g Vinylpyrrolidon und 240 g Wasser. Als Zulauf 2 diente eine Lösung von 2,4 g 2,2'-Azobis-(2-amidinopropan)dihydrochlorid in 75 g Wasser. In einem 2 l-Glasgefäß, das mit Rührer, Heizung, Rückflußkühler, Gasein- und auslaß und Dosiervorrichtungen ausgestattet war, wurden 200 g Wasser, 100 ml Zulauf 1 und 10 ml Zulauf 2 vorgelegt und auf 60°C erwärmt. Dann wurden bei dieser Temperatur der restliche Zulauf 1 in 5 Stunden und der restliche Zulauf 2 in 7 Stunden zudosiert und noch eine Stunde nachgerührt. Man erhielt nach Verdünnen mit 700 ml Wasser eine klare, viskose Polymerlösung mit einem Feststoffgehalt von 24,8 %. Der K-Wert der erhaltenen Polymerlösung betrug 124,2 (0,1 %ig in Wasser).

### Beispiel 27

Beispiel 6 wurde mit der Abänderung wiederholt, daß man Maltodextrin wegließ. Man erhielt eine klare, viskose Polymerlösung mit einem Feststoffgehalt von 23,2 %. Der K-Wert der erhaltenen Lösung lag bei 127,3 (0,1 %ig in Wasser).

### Beispiel 28

Beispiel 22 wurde mit den Abänderungen wiederholt, daß man die gesamte Saccharose, 360 ml Wasser aus Zulauf 1 und 120 ml Wasser aus der Vorlage wegließ. Man erhielt eine hochviskose Polymerlösung mit einem Feststoffgehalt von 20,3 % und einem K-Wert von 291,3 (0,1 %ig in Wasser).

### Beispiel 29

Dieses Beispiel entspricht Beispiel 2 aus der Literaturstelle (3). Man erhielt eine hochviskose Polymerlösung mit einem Feststoffgehalt von 5 20,2 %. Der K-Wert betrug 299,7 (0,1 %ig in Wasser).

### Beispiel 30

Dieses Beispiel entspricht Beispiel 6 aus der Literaturstelle (2). Man 0 erhielt eine gelbliche, viskose Polymerlösung mit einem Feststoffgehalt von 21,2 %. Der K-Wert betrug 286,3 (0,1 %ig in Wasser).

### Mischungen: Beispiele 31 bis 54

### Beispiele 31 bis 36

Eine Polymerlösung, hergestellt gemäß Beispiel 25, wurde mit Saccharose in unterschiedlichen Verhältnissen gemischt. Ebenso wurde eine Lösung gemäß Beispiel 27 mit Saccharose gemischt.

**Tabelle 1**

| Beispiel Nr. | 31 | 32 | 33 | 34 | 35 | 36 |
|---|---|---|---|---|---|---|
| Gew.-Verhältnis Polymer/Saccharose | 2:1 | 1:1 | 1:2 | 2:1 | 1:1 | 1:2 |
| Festgehalt [%] | 19,9 | 19,7 | 19,1 | 39,4 | 39,2 | 35,5 |
| K-Wert (0,1 %ig in H₂O) | 254,5 | 243,8 | 238,2 | 161,3 | 149,7 | 137,1 |
| Polymer aus Bsp. | 25 | 25 | 25 | 27 | 27 | 27 |

### Beispiele 37 bis 42

Beispiele 31 bis 36 wurden jeweils mit der Abänderung wiederholt, daß man anstelle von Saccharose die gleiche Menge Maltodextrin mit einem DE-Wert (nach Luff-Schoorl) von 11 bis 14 einsetzte.

**Tabelle 2**

| Beispiel Nr. | 37 | 38 | 39 | 40 | 41 | 42 |
|---|---|---|---|---|---|---|
| Gew.-Verhältnis Polymer/Maltodextrin | 2:1 | 1:1 | 1:2 | 2:1 | 1:1 | 1:2 |
| Festgehalt [%] | 19,2 | 19,6 | 19,3 | 38,9 | 38,3 | 38,8 |
| K-Wert (0,1 %ig in H₂O) | 257,2 | 244,2 | 232,0 | 163,4 | 151,7 | 134,3 |
| Polymer aus Bsp. | 25 | 25 | 25 | 27 | 27 | 27 |

### Beispiele 43 bis 45

Ein Polymer gemäß Beispiel 29 wurde mit unterschiedlichen Mengen Saccharose gemischt.

**Tabelle 3**

| Beispiel Nr. | 43 | 44 | 45 |
|---|---|---|---|
| Gew.-Verhältnis Polymer/Saccharose | 2:1 | 1:1 | 1:2 |
| Festgehalt [%] | 19,3 | 19,7 | 20,7 |
| K-Wert (0,1 %ig in H₂O) | 288,3 | 278,4 | 270,9 |

### Beispiele 46 bis 48

Ein Polymer gemäß Beispiel 29 wurde mit unterschiedlichen Mengen Maltodextrin mit einem DE-Wert (nach Luff-Schoorl) von 11 bis 14 gemischt.

**Tabelle 4**

| Beispiel Nr. | 46 | 47 | 48 |
|---|---|---|---|
| Gew.-Verhältnis Polymer/Maltodextrin | 2:1 | 1:1 | 1:2 |
| Festgehalt [%] | 20,5 | 21,8 | 19,0 |
| K-Wert (0,1 %ig in H₂O) | 289,3 | 276,4 | 264,3 |

### Beispiele 49 bis 51

Ein Polymer gemäß Beispiel 30 wurde mit unterschiedlichen Mengen Saccharose gemischt.

**Tabelle 5**

| Beispiel Nr. | 49 | 50 | 51 |
|---|---|---|---|
| Gew.-Verhältnis Polymer/Saccharose | 2:1 | 1:1 | 1:2 |
| Festgehalt [%] | 20,7 | 20,0 | 19,6 |
| K-Wert (0,1 %ig in H₂O) | 266,3 | 254,2 | 251,0 |

### Beispiele 52 bis 54

Ein Polymer gemäß Beispiel 30 wurde mit unterschiedlichen Mengen Maltodextrin mit einem DE-Wert (nach Luff-Schoorl) von 11 bis 14 gemischt.

**Tabelle 6**

| Beispiel Nr. | 52 | 53 | 54 |
|---|---|---|---|
| Gew.-Verhältnis Polymer/Maltodextrin | 2:1 | 1:1 | 1:2 |
| Festgehalt [%] | 20,0 | 19,0 | 21,2 |
| K-Wert (0,1 %ig in H₂O) | 269,4 | 256,0 | 253,2 |

In Tabelle 7 werden die Eigenschaften der erfindungsgemäßen Polymerlösungen gemäß den Beispielen 1 bis 24 mit denen der Zubereitungen verglichen, die zum Vergleich gemäß den Beispielen 25 bis 54 hergestellt wurden.

Verglichen wurden die Eigenschaften Wasseraufnahme, Anionentensidverträglichkeit, Geruch und Farbe.

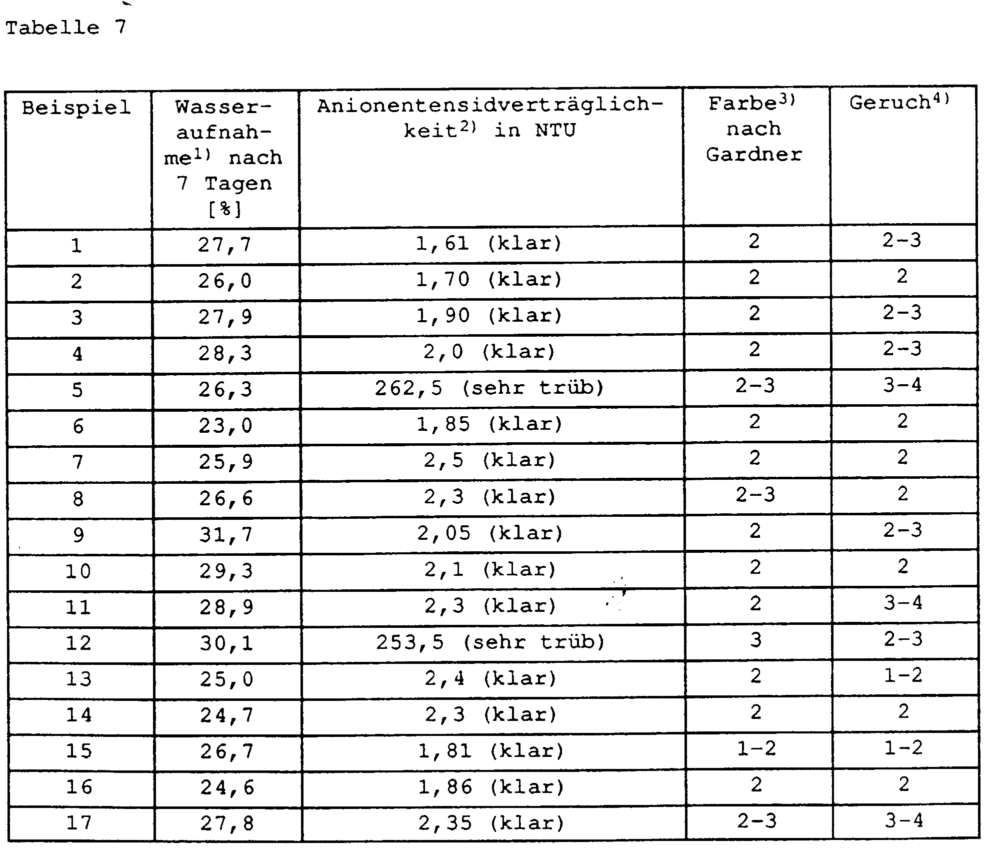

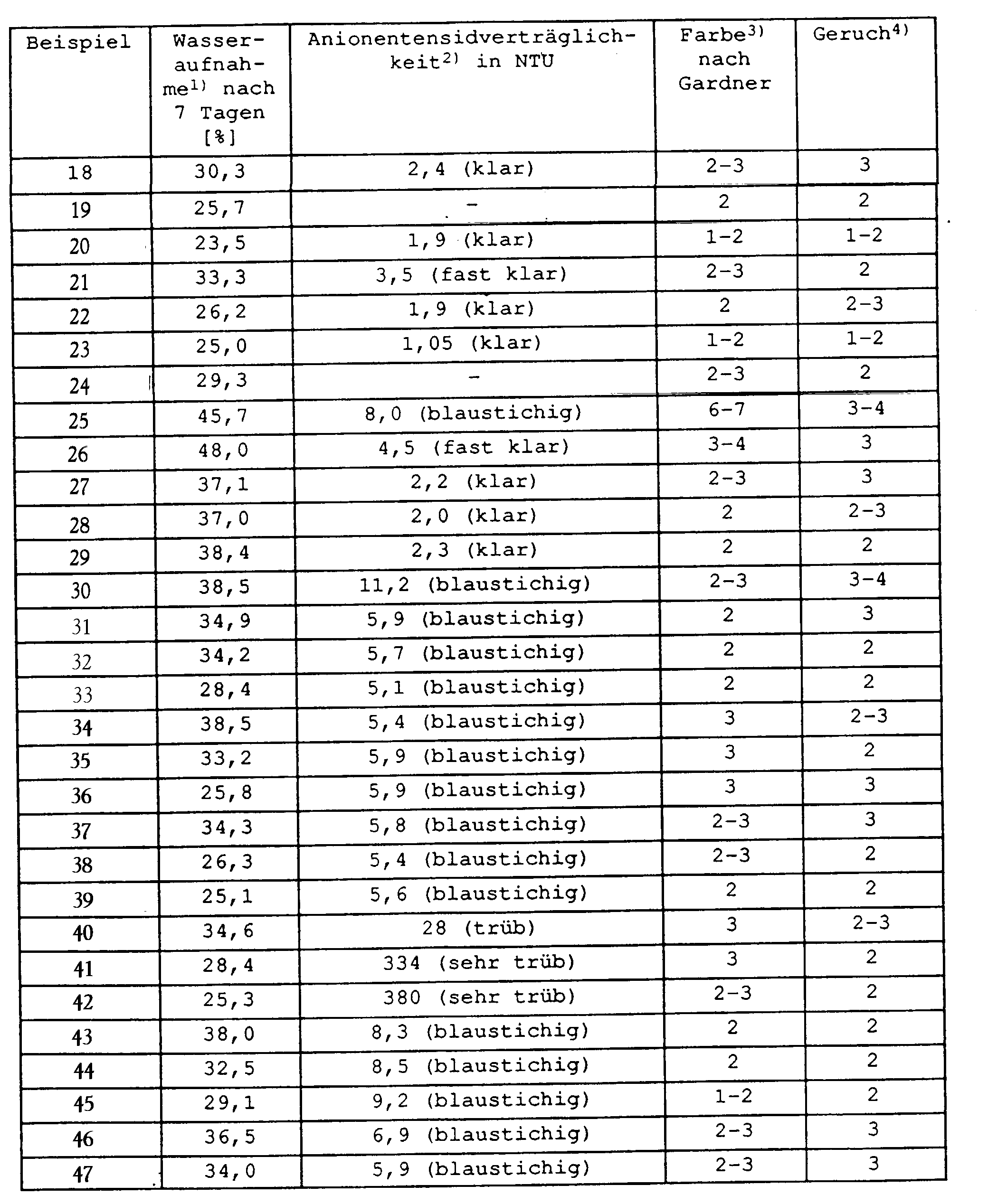

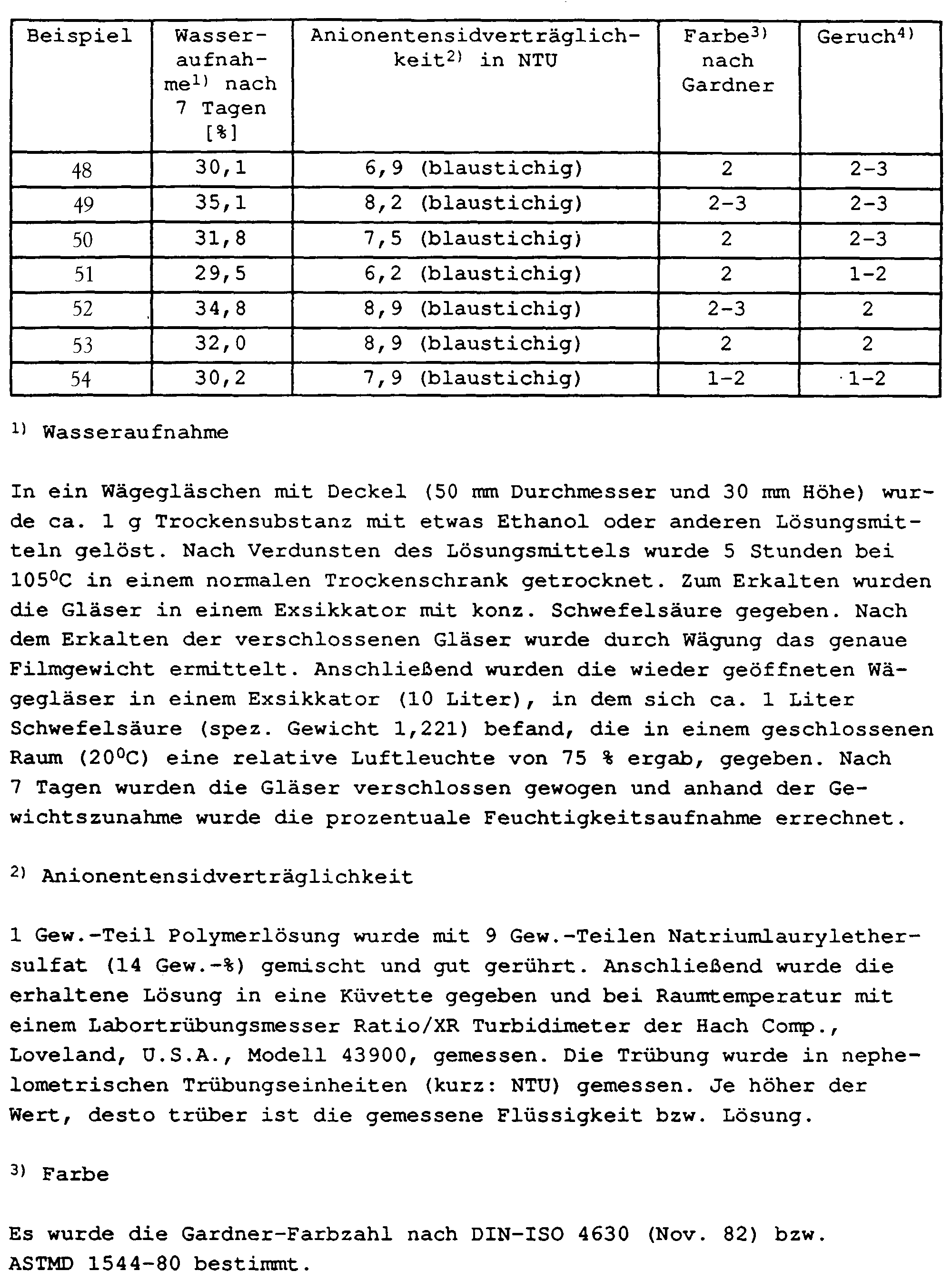

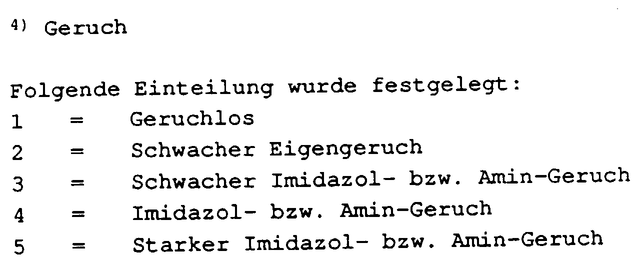

### Anwendungsbeispiele

Formulierugen für Haarkonditioniermittel:

### Beispiele 55 bis 58

### Beispiel 55 (Zum Vergleich)

| | |
|---|---|
| 2,0 % | des Polymers aus Beispiel 25 (bez. auf die Festsubstanz) |
| 0,5 % | Cetyl-hydroxyethyl-dimethylammonium-dihydrogenphosphat |
| 1,0 % | Copolymerisat aus 60 % Vinylpyrrolidon und 40 % Vinylacetat |
| 0,1 % | eines handelsüblichen Parfüms |
| 0,3 % | eines handelsüblichen Solubilisators |
| 0,2 % | eines handelsüblichen Konservierungsmittels auf Basis von 2-Methyl-4-isothiazolin-3-on |
| 85,9% | destilliertes Wasser |
| 10,0% | Propan/Butan (25 %/75 %) |

Die Wirksubstanz wurde klar gelöst. Die Festigungswirkung auf dem Haar war gut, aber der erhaltene Schaum war cremig.

### Beispiel 56

Die Zusammensetzung entsprach der Formulierung von Beispiel 55 mit dem Unterschied, daß anstelle des Polymers aus Beispiel 25 die gleiche Menge des Polymers aus Beispiel 10 verwendet wurde.

Die Wirksubstanz wurde klar gelöst, die Festigungswirkung auf dem Haar war gut und man erhielt einen schönen, trockenen Schaum.

### Beispiel 57

Die Zusammensetzung entsprach der Formulierung von Beispiel 55 mit dem Unterschied, daß anstelle des Polymers aus Beispiel 25 die gleiche Menge des Polymers aus Beispiel 14 verwendet wurde.

Die Wirksubstanz wurde klar gelöst, die Festigungswirkung auf dem Haar war sehr gut und man erhielt einen schönen, trockenen Schaum.

### Beispiel 58

Die Zusammensetzung entsprach der Formulierung von Beispiel 55 mit dem Unterschied, daß anstelle von 2,0 % des Polymers aus Beispiels 25 2,5 % des Polymers aus Beispiel 15 und entsprechend weniger destilliertes Wasser verwendet wurden.

Die Wirksubstanz wurde klar gelöst, die Festigungswirkung auf dem Haar war sehr gut und man erhielt einen schönen, trockenen Schaum.

Formulierungen für Fönlotionen als Conditioner und Festiger:

### Beispiele 59 bis 62

### Beispiel 59 (Zum Vergleich)

| | |
|---|---|
| 1,0 % | des Polymers aus Beispiel 25 |
| 0,5 % | Cetyl-hydroxyethyl-dimethylammonium-dihydrogenphosphat |
| 1,0 % | Copolymerisat aus 60 % Vinylpyrrolidon und 40 % Vinylacetat |
| 0,1 % | eines handelsüblichen Parfüms |
| 0,3 % | eines handelsüblichen Solubilisators |
| 20,0% | Ethanol |
| 77,1% | destilliertes Wasser |

Die Festigungswirkung auf dem Haar war gut. Die Wirksubstanz war klar gelöst.

### Beispiel 60

Die Zusammensetzung entsprach der Formulierung von Beispiel 59 mit dem Unterschied, daß anstelle des Polymers aus Beispiel 25 die gleiche Menge des Polymers aus Beispiel 10 verwendet wurde.

Die Festigungswirkung auf dem Haar war gut bis sehr gut. Die Wirksubstanz war klar gelöst.

### Beispiel 61

Die Zusammensetzung entsprach der Formulierung von Beispiel 59 mit dem Unterschied, daß anstelle des Polymers aus Beispiel 25 die gleiche Menge des Polymers aus Beispiels 14 verwendet wurde.

Die Festigungswirkung auf dem Haar war sehr gut. Die Wirksubstanz war klar gelöst.

### Beispiel 62

Die Zusammensetzung entsprach der Formulierung von Beispiel 59 mit dem Unterschied, daß anstelle des Polymers aus Beispiel 25 die gleiche Menge des Polymers aus Beispiel 15 verwendet wurde.

Die Festigungswirkung auf dem Haar war sehr gut. Die Wirksubstanz war klar gelöst.

### Schaumwirkung: Beispiel 63

In einem 1 1-Becherglas (hohe Form) wurde eine Lösung von 0,5 g Natrium-Laurylethersulfat in 600 ml Wasser mit 1,0 g des jeweiligen Polymers (bez. auf die Festsubstanz) - außer bei der Blindprobe - versetzt und bei 25°C mit einem Flügelrührer (ø 5 cm) 1 min bei 1400 Upm gerührt. 30 sec nach Abstellen des Rührers wurde die Schaumhöhe abgelesen (1. Wert). Danach wurden weitere 3 min gerührt. Wiederum 30 sec nach Abstellen des Rührers wurde die Schaumhöhe erneut gemessen (2. Wert).

Die Ergebnisse sind in Tabelle 8 wiedergegeben.

**Tabelle 8**

| Schaumwirkung | | |
|---|---|---|
| Polymer aus Beispiel | Schaumhöhe [cm] | |
| | 1. Wert | 2. Wert |
| - (Blindprobe) | 2,6 | 3,8 |
| 25 (Zum Vergleich) | 1,6 | 3,2 |
| 10 | 1,2 | 3,6 |
| 14 | 1,8 | 3,6 |
| 15 | 2,6 | 3,5 |

Bei allen Meßversuchen war die Wasserphase klar.

### Formulierung für Haarshampoos: Beispiel 64

| | |
|---|---|
| 40,0 % | Natrium-Laurylethersulfat |
| 10,0 % | Cocamido-propyl-betain |
| 10,0% | hydrolysierte tierische Proteine |
| 5,0 % | des Polymers aus Beispiel 10 oder 14 |
| 1,0 % | eines handelsüblichen Solubilisators |
| 0,05% | eines handelsüblichen UV-stabilisators auf Basis eines Benzophenon-Derivates |
| 0,1 % | eines handelsüblichen Konservierungsmittels auf Basis von 2-Methyl-4-isothiazolin-3-on |
| 0,8 % | Natriumchlorid |
| ad 100 % | Wasser |

Bei der getesteten Rezeptur zeigten beide Polymere ein gutes Verhalten als Shampoo.

### Formulierung für Haarpackungen: Beispiel 65

| | |
|---|---|
| 3,0 % | handelsübliche Emulgatoren |
| 4,0 % | Cetylstearylalkohol |
| 2,0 % | Cetearyloctanoat |
| 0,1 % | Siliconöl |
| 5,0 % | des Polymers aus Beispiel 10, 14 oder 15 |
| 2,0 % | Panthenol |
| 2,5 % | Propylenglykol |
| 0,1 % | eines handelsüblichen Konservierungsmittels |
| ad 100 % | Wasser |

Alle drei Polymere konnten in Emulsion eingesetzt werden. Nach 6 Wochen Lagerung bei 6°C, 25°C und auch bei 43°C konnte in keinem Fall eine Veränderung der Formulierung festgestellt werden.

### Beispiel 66

760 g eines Stärkeverzuckerungsproduktes mit einem DE-Wert von 17 bis 19,9 wurden in 570 g Wasser gelöst, dann wurden 190 g N-Vinylpyrrolidon und 2 g einer 75 gew.-%igen Lösung von t-Butylperpivalat in Kohlenwasserstoffen (Luperox 11 der Fa. Pennwalt) zugesetzt. Nach Erhitzen der Mischung auf 75°C wurden gleichzeitig innerhalb von zwei Stunden 570 g N-Vinylpyrrolidon und innerhalb von drei Stunden 10 g einer 75 %igen t-Butylperpivalatlösung in 100 g Ethanol zugetropft. Anschließend wurde nach Zugabe von weiteren 10 g der t-Butylperpivalatlösung in 100 g Ethanol die Temperatur auf 90°C erhöht und weitere zwei Stunden gerührt. Nach dem Abtrennen des Ethanols durch Wasserdampfdestillation wurde die Mischung mit 500 g Wasser verdünnt. Der Feststoffgehalt der schwach gelben, trüben Lösung lag bei 40,6 %, der K-Wert des Polymeren bei 46 (1 %ig in Wasser).

### Herstellung des Iodkomplexes:

285 g der so erhaltenen Polymerlösung wurden mit 468 g Wasser verdünnt, dann wurden 1,3 g Ameisensäure und 28 g Iod zugesetzt. Anschließend wurde eine Stunde bei Raumtemperatur und 20 Stunden bei 70°C gerührt. Man erhielt eine Iodkomplexlösung mit einem Feststoffgehalt von 17,6 % und einem verfügbaren (mit Thiosulfatlösung titrierbaren) Iodgehalt von 11,7 % (bezogen auf Feststoff). Der Gehalt an nicht komplexiertem Iod (bestimmt durch Dialyse) in einer auf 1 % an verfügbarem Iodgehalt verdünnten Lösung lag bei nur 0,0007 %. Die verdünnte Lösung zeichnete sich durch gute Lagerstabilität aus. Sie verlor nach 15 Stunden Lagerung bei 80°C nur 2 % ihres Gehalts an verfügbarem Iod.

### Anwendung als Farbübertragungsverhinderer

Die Farbstoffübertragung von farbigen Textilien auf weiße oder hellfarbige Textilien während des gemeinsamen Waschens ist ein seit langem bekanntes Problem, das durch den Trend zur gemeinsamen Wäsche (Buntwäsche) zunehmend an Bedeutung gewinnt. Zur Verhinderung der Farbstoffübertragung wird die Verwendung von Waschmitteln mit farbstoffübertragungsinhibierenden Eigenschaften empfohlen.

Die Patentliteratur beschreibt Wasch- und Reinigungsmittel, deren farbstoffübertragungsinhibierende Wirkung auf dem Zusatz von Homo- bzw. Copolymeren des N-Vinylpyrrolidons (NVP) oder des N-Vinylimidazols (NVI) beruht (DE-OS 22 32 353, DE-OS 28 14 287). Nachteil der Polymeren auf NVP-Basis ist ihre zu geringe Wirkung gegenüber einer Anzahl von Ausfärbungen. Polymere auf NVI-Basis sind wirksamer, neigen jedoch zur Ausbildung von sehr unangenehmen Gerüchen. Gemeinsam ist diesen Polymeren der Nachteil, daß sie weder biologisch abbaubar sind noch durch Klärschlammadsorption aus dem Abwasser entfernt werden können.

### Allgemeine Bemerkungen zu den Waschversuchen

Zur Prüfung der Wirksamkeit wurde weißes Prüfgewebe gemeinsam mit eingefärbten Textilproben aus Baumwolle im Launderometer gewaschen. Die Messung der aufgetretenen Farbstoffübertragung erfolgte photometrisch.

Aus den an den einzelnen Prüfgeweben gemessenen Remissionswerten wurden die jeweiligen Farbstärken bestimmt, aus denen sich die Wirksamkeit der Polymeren ableiten läßt. Eine Wirksamkeit von 100 % bedeutet, daß das Testgewebe seine ursprüngliche Farbstärke beibehielt, es also nicht angefärbt wurde. Eine Wirksamkeit von 0 % wird bei einem prüfgewebe ermittelt, das die gleiche Farbstärke aufweist wie ein Prüflappen der ohne Zusatz eines farbstoffübertragungsverhindernden Additivs gewaschen wurde.

Die Textilproben wurden mit folgenden Farbstoffen gefärbt:

Direkt Schwarz 51 (C.I. 27720), Direkt Blau 218 (C.I. 24401), Direkt Rot 79 (C.I. 29065), Direkt Schwarz 22 (C.I. 35435), Direkt Blau 71 (C.I. 34140) und Reaktiv Schwarz 5 (C.I. 20505).

| Waschbedingungen | |
|---|---|
| Waschgerät | Launderometer |
| Waschzyklen | 1 |
| Temperatur | 60°C |
| Waschdauer | min |
| Wasserhärte | mmol Ca²⁺, Mg²⁺ (4:1)/1 |
| Prüfgewebe | Baumwolle |
| Flottenverhältnis | 1:50 ml |
| Flottenmenge | 250 ml |
| Waschmittelkonzentration | 7,0 g/l |

| Waschmittelzusammensetzung [%] | |
|---|---|
| Zeolith A | 20 |
| Natriumcarbonat | 11 |
| lin. Dodecylbenzolsulfonat | 5 |
| Seife | 1,3 |
| C₁₃/C₁₅-Oxoalkohol x 7 Ethylenoxid-Einheiten | 3,9 |
| Acrylsäure/Maleinsäure-Copolymerisat 70/30, Na-Salz, MG = 70000 | 2,7 |
| Na-Carboxymethylcellulose | 0,4 |
| Wasser | 7,0 |
| Polymer 1, 2 oder 3 | 1,0 |
| Natriumsulfat | 100 |

- Polymer 1: Polyvinylpyrrolidon, K-Wert 30 (1 %ig in H₂O), Standardpolymer zum Vergleich
- Polymer 2: Synthese nach der im Beispiel 72 angegebenen Vorschrift
- Polymer 3: Synthese nach der im Beispiel 71 angegebenen Vorschrift

### Ergebnis

| | Wirksamkeit [%] | | |
|---|---|---|---|
| | Polymer 1 | Polymer 2 | Polymer 3 |
| Direkt Schwarz 51 | 35 | 36 | 28 |
| Direkt Blau 218 | 38 | 42 | 100 |
| Direkt Rot 79 | 83 | 86 | 87 |
| Direkt Schwarz 22 | 81 | 87 | 91 |
| Direkt Blau 71 | 98 | 99 | 98 |
| Reaktiv Schwarz 5 | 17 | 13 | 46 |

Die Ergebnisse zeigen, daß die in Gegenwart von Sacchariden hergestellten Polymeren 2 und 3 (Saccharidanteil 30 bzw. 50 %) nicht nur an die Wirkung von Polyvinylpyrrolidon heranreichen, sondern sie sogar übertreffen. Saccharide alleine sind unwirksam.

### Synthesebeispiele

### Beispiel 67

In einer Rührapparatur mit aufgesetztem Rückflußkühler wurde ein Gemisch aus 95 g N-Vinylimidazol, 380 g abgebauter Stärke (DE-Wert nach Luff-Schoorl: 17-20) und 285 g Wasser vorgelegt und unter Zugabe von 1,1 g t-Butylperoxy-2-ethylhexanoat im Stickstoffstrom auf 85°C erhitzt. Nach Erreichen dieser Temperatur wurden Zulauf 1 (8285 g N-Vinylimidazol) innerhalb von 2 h und Zulauf 2 (5,1 g t-Butylperoxy-2-ethylhexanoat in 50 ml Ethanol) innerhalb von 3 h zugetropft. Anschließend wurde eine Lösung von 5,7 g t-Butylperoxy-2-ethylhexanoat in 25 ml Ethanol zugegeben und das Reaktionsgemisch 1 h auf 95°C erhitzt. Nach beendeter Reaktion wurde abgekühlt und mit 300 ml Wasser verdünnt. Man erhielt eine bräunliche, klare, viskose Flüssigkeit, die keinen unangenehmen Geruch aufwies. Der K-Wert (1 %ig in Wasser) betrug 41,4.

### Beispiel 68

In einer Rührapparatur mit aufgesetztem Rückflußkühler wurde ein Gemisch aus 50 g N-Vinylpyrrolidon, 120 g Saccharose und 280 ml Wasser vorgelegt und unter Zugabe von 1,20 g 2,2'-Azobis(2-amidinopropan)dihydrochlorid im Stickstoffstrom auf 60°C erhitzt. Nach Erreichen dieser Temperatur wurde Zulauf 1 (230 g VP in 250 ml Wasser) innerhalb von 5 h und Zulauf 2 (2,20 g 2,2'-Azobis(2-amidinopropan)dihydrochlorid in 70 ml Wasser) innerhalb von 6 h zudosiert. Anschließend wurde 2 h bei 60°C nachpolymerisiert. Man erhielt eine hellbraune, klare, viskose Flüssigkeit mit einem K-Wert von 54 (0,1 %ig in Wasser).

## Patentansprüche

1. Verfahren zur Herstellung von Polymerisaten aus ethylenisch ungesäztigten Verbindungen, die mindestens ein kovalent gebundenes N-Atom im Molekül enthalten, pclymerisiert in Gegenwart von Monosacchariden, Oligosacchariden, Polygosacchariden, oder deren Derivaten, dadurch gekennzeichnet, daß man
(A) Monomere oder Monomerenmischungen aus der Gruppe
(a) N-Vinylimidazole, welche am heterocyclischen Ring durch bis zu drei C₁- bis C₁₂-Alkylreste substituiert sein und in N-quaternisierter Form oder in Salzfcrm vorliegen können,
(b) fünf- bis achtgliedrige N-Vinyllactame, welche am Ring durch bis zu drei C₁- bis C₁₂-Alkylreste substituiert sein können,
(c) Acrylsäure- oder Methacrylsäure-dialkylamnoalkylester mit insgesamt bis zu 30 C-Atomen im Dialkylaminoalkyl-Rest, welche in N-quaternisierter Form oder in Salzform vorliegen können und
(d) N-(Dialkylaminoalkyl)-acrylsäure- oder -methacrylsäureamide mit insgesamt bis zu 30 C-Atomen in Dialkylaminoalkyl-Rest, welche in N-quaternisierter Form oder in Salzform vorliegen können,
wobei als weitere Comonomere zu (A) noch
(f) monoethylenisch ungesättigte C₃- bis C₁₀-Carbonsäuren und deren Alkalimetall-, Erdalkalimetall- oder Ammoniumsalze,
(g) monoethylenisch ungesättigte C₃- bis C₁₀-Carbonsäureester sowie
(h) in geringen Mengen mindestens zwei ethylenisch ungesättigte, nicht konjugierte Doppelbindungen im Molekül enthaltende Verbindungen vorhanden sein können, wobei entweder die Verbindungen (a) oder (b) jeweils allein oder Mischungen aus den Verbindungen (a) bis (d) gewünschtenfalls mit (f) bis (h) eingesetzt werden und wobei (h) maximal in einer Menge bis zu 5 Gew.-%, bezogen auf die Gesamtmenge aller Monomeren (A), verwendet wird, in Gegenwart von
(B) Monosacchariden, Oligosacchariden, Polysacchariden, thermisch oder mechanisch behandelten, oxidativ, hydrolytisch oder enzymatisch abgebauten Polysacchariden, oxidierten hydrolytisch oder enzymatisch abgebauten Polysacchariden, chemisch modifizierten Mono-, Oligo- und Polysacchariden oder Mischungen der genannten Verbindungen (B)
im Gewichtsverhältnis (A):(B) von (95 bis 20):(5 bis 80) in wäßrigen Systemen durch Lösungspolymerisation radikalisch initiiert polymerisiert.

2. Verfahren nach Anspruch 1, dadurch gekennzeichnet, daß man als Monomere (A) entweder die Verbindungen (a) oder (b) jeweils allein oder Mischungen aus den Verbindungen (a) und (b) einsetzt.

3. Verfahren nach Anspruch 1 oder 2, dadurch gekennzeichnet, daß man als Komponente (B) Stärken, thermisch und/oder mechanisch behandelte Stärken, oxidativ, hydrolytisch oder enzymatisch abgebaute Stärken, oxidierte hydrolytisch oder oxidierte enzymatisch abgebaute Stärken oder chemisch modifizierte Stärken einsetzt.

## Claims

1. A process for the preparation of a polymer of ethylenically unsaturated compounds which contain one or more covalently bonded nitrogen atoms in the molecule, polymerized in the presence of monosaccharides, oligosaccharides, polysaccharides or derivatives thereof, wherein
(A) monomers or monomer mixtures selected from the group consisting of
(a) N-vinylimidazoles which may be substituted in the heterocyclic ring by not more than three C₁-C₁₂-alkyl radicals and may be present in the N-quaternized form or in salt form,
(b) five-membered to eight-membered N-vinyllactams which may be substituted in the ring by not more than three C₁-C₁₂-alkyl radicals,
(c) dialkylaminoalkyl acrylates or methacrylates where the dialkylaminoalkyl radical has a total of not more than 30 carbon atoms and which may be present in the N-quaternized form or in salt form and
(d) N-(dialkylaminoalkyl)-acrylamides or -methacrylamides where the dialkylaminoalkyl radical has a total of not more than 30 carbon atoms and which may be present in the N-quaternized form or in salt form,
further comonomers (A) which may be present being
(f) monoethylenically unsaturated C₃-C₁₀-carboxylic acids and alkali metal, alkaline earth metal or ammonium salts thereof,
(g) monoethylenically unsaturated C₃-C₁₀-carboxylates and
(h) small amounts of compounds containing two or more ethylenically unsaturated, nonconjugated double bonds in the molecule,
with either the compounds (a) or (b) being used alone in each case or a mixture of the compounds (a) to (d), if desired with (f) to (h), being used, and with (h) being used in an amount of not more than 5% by weight, based on the total weight of all the monomers (A), are subjected to free radical solution poly- merization in aqueous systems in the presence of
(B) monosaccharides, oligosaccharides, polysaccharides, thermally or mechanically treated, oxidatively, hydrolytically or enzymatically degraded polysaccharides, oxidized hydrolytically degraded or enzymatically degraded polysaccharides, chemically modified mono-, oligo- or polysaccharides or a mixture of the stated compounds (B)
in a weight ratio (A) : (B) of (95 to 20) : (5 to 80).

2. A process as claimed in claim 1, which comprises using as monomers (A) either the compounds (a) or (b) alone in each case or a mixture of the compounds (a) and (b).

3. A process as claimed in claim 1 or 2, which comprises using as component (B) starches, thermally and/or mechanically treated starches, oxidatively, hydrolytically or enzymatically degraded starches, oxidized hydrolytically degraded or oxidized enzymatically degraded starches or chemically modified starches.

## Revendications

1. Procédé de préparation de polymères constitués de composés éthyléniquement insaturés qui contiennent au moins un atome d'azote lié par covalence dans la molécule, polymérisés en présence de monosaccharides, d'oligosaccharides, de polysaccharides ou de leurs dérivés, caractérisé en ce que l'on polymérise dans des systèmes aqueux par polymérisation en solution amorcée par voie radicalaire,
(A) des monomères ou des mélanges de monomères choisis parmi le groupe
(a) des N-vinylimidazoles, qui sont substitués sur le noyau hétérocyclique par jusqu'à trois radicaux alkyle en C₁ à C₁₂ et peuvent se présenter sous la forme N-quaternisée, ou sous la forme de sel,
(b) des N-vinyllactames penta à octogonaux, qui peuvent être substitués sur le noyau par jusqu'à trois radicaux alkyle en C₁ à C₁₂.
(c) des esters dialkylaminoalkyliques de l'acide acrylique ou de l'acide méthacrylique, avec au total jusqu'à 30 atomes de carbone dans le reste dialkylaminoalkyle, qui peuvent se présenter sous la forme N-quaternisée ou sous la forme de sel et
(d) des amides des acides N-(dialkylaminoalkyl)-acryliques ou des acides N- (dialkylaminoalkyl)-méthacryliques, avec au total jusqu'à 30 atomes de carbone dans le groupe dialkylaminoalkyle, qui peuvent se présenter sous la forme N-quaternisée ou sous la forme de sel,
où peuvent encore être présents, à titre d'autres comonomères en plus de (A), encore
(f) des acides carboxyliques en C₃ à C₁₀ monoéthyléniquement insaturés et leurs sels de métaux alcalins, de métaux alcalino-terreux ou d'ammonium,
(g) des esters d'acides carboxyliques en C₃ à C₁₀ monoéthyléniquement insaturés, ainsi que
(h) en faibles proportions, des composés contenant au moins deux doubles liaisons éthyléniquement insaturées et non conjuguées dans la molécule,
où les composés (a) ou (b) s'utilisent à chaque fois seuls, ou bien on utilise des mélanges des composés (a) à (d), le cas souhaité avec (f) à (h) et où (h) s'utilise au maximum en une proportion allant jusqu'à 5% en poids, par rapport à la quantité totale de tous les monomères (A), en présence de
(B) des monosaccharides, des oligosaccharides, des polysaccharides, des polysaccharides thermiquement ou mécaniquement traités, dégradés par voie oxydante, hydrolytique ou enzymatique, des polysaccharides oxydés, dégradés par voie hydrolytique ou enzymatique, des monosaccharides, des oligosaccharides et des polysaccharides, chimiquement modifiés, ou des mélanges des composés (B) susmentionnés,
dans le rapport pondéral (A) : (B) de (95 à 20) : (5 à 80) .

2. Procédé suivant la revendication 1, caractérisé en ce que l'on utilise, à titre de monomères (A), soit les composés (a) ou (b) à chaque fois seuls, ou des mélanges des composés (a) et (b).

3. Procédé suivant la revendication 1 ou 2, caractérisé en ce que l'on utilise, à titre de composant (B), des amidons, des amidons thermiquement et/ou mécaniquement traités, des amidons dégradés par voie oxydante, hydrolytique ou enzymatique, des amidons oxydés, dégradés par voie hydrolytique ou des amidons oxydés, dégradés par voie enzymatique, ou des amidons chimiquement modifiés.
